# EUROPEAN PATENT APPLICATION

(11) **EP 4 578 946 A1**
(43) Date of publication of application: **02.07.2025**
(21) Application number: 23864403.3
(22) Date of filing: 03.07.2023
(51) Int. Cl.: C12N 9/22, C12N 15/55, C12N 15/113, C12N 15/63

(54) **OPTIMIZED GUIDE RNA, CRISPR/ACC2C9 GENE EDITING SYSTEM, AND GENE EDITING METHOD**

(30) Priority: 15.09.2022 CN 202211124835
(71) Applicant: Shanghaitech University, Shanghai 201210 (CN)
(72) Inventor: JI, Quanjiang, Shanghai 201210 (CN); MA, Jiacheng, Shanghai 201210 (CN); CHEN, Weizhong, Shanghai 201210 (CN)
(74) Representative: Canzler & Bergmeier Patentanwälte Partnerschaft mbB
(86) International application number: PCT/CN2023/101339
(87) International publication number: WO 2024/055664

(57) **Abstract**

The guide RNA in the present disclosure comprises an RNA framework and a gene targeting section. The RNA framework comprises tracrRNA and a tracr duplex. A nucleotide sequence of the tracrRNA is a sequence obtained by addition, deletion, or substitution of one or more nucleotides in a nucleotide sequence set forth in SEQ ID NO. 117. A nucleotide sequence of the tracr duplex is a sequence obtained by addition, deletion, or substitution of one or more nucleotides in a nucleotide sequence set forth in SEQ ID NO. 127. By utilizing the guide RNA and the CRISPR/AcC2C9 gene editing system, efficient and accurate gene editing in a cell can be realized. Compared with a traditional CRISPR/AcC2C9 gene editing system, the editing efficiency and applicability of the editing system in the present disclosure can be greatly improved.

## Description

### Background of the Present Disclosure

### FIELD OF DISCLOSURE

The present disclosure relates to the technical field of biology, and particularly, to a guide RNA, a CRISPR/AcC2C9 gene editing system, and a gene editing method.

### DESCRIPTION OF RELATED ARTS

Clustered regularly interspaced short palindromic repeats (CRISPR) and the associated proteins (Cas), initially discovered as adaptive immune systems in archaea and bacteria, have now developed into multifunctional genome editing tools due to the nuclease activity of their effector proteins. A guide RNA directs a Cas nuclease to specific targets in the genome of multiple cells, then the Cas nuclease performs cleavage to cause DNA double-stranded breakage, and later the endogenous or exogenous DNA repair mechanism of the cell is carried out, thus achieving the target gene modification. Genomic editing technology can promote gene engineering, cell engineering, the construction of model animals, plant gene screening, etc., and has great potential in the treatment of human diseases.

At present, CRISPR/Cas9 and CRISPR/Cas12a are the two most widely used genome editing systems. However, these two systems, both of which are large proteins of more than 1000 amino acids, have a large size. It is known that such a large molecular weight is difficult to deliver, thus limiting their use in the field of disease treatment. Recently, a Cas protein with a small size, namely C2C9, which consists of about 400-700 amino acid residues and contains a RuvC nuclease domain, has been discovered. Such small-sized proteins can help solve the problem of load limitation of adeno-associated virus vectors. It was reported that *Actinomadura craniella* C2C9 (AcC2C9, 506 amino acids) can target the target gene in eukaryotic cells and realize gene insertion and deletion with an editing efficiency of only 0.1%-25%. Therefore, it is particularly urgent to optimize the AcC2C9 gene editing system and make it an efficient and accurate gene editing tool.

### SUMMARY OF THE PRESENT DISCLOSURE

The present disclosure provides a guide RNA, a CRISPR/AcC2C9 gene editing system and a gene editing method. The nuclease with a small size in the present disclosure has a high editing efficiency. By utilizing the optimized gene editing system and method provided by the present disclosure, efficient and accurate editing of target genes or target genomes can be realized *in-vitro* or *in-vivo* (including within cells), and the gene editing efficiency of the small-sized CRISPR/AcC2C9 system is greatly improved.

The present disclosure provides a guide RNA, comprising an RNA framework and a gene targeting section, the RNA framework includes a tracrRNA and a tracr duplex linked directly or through a linker; a nucleotide sequence of the tracrRNA is a sequence obtained by addition, deletion, or substitution of one or more nucleotides in a nucleotide sequence set forth in SEQ ID NO. 117, and a nucleotide sequence of the tracr duplex is a sequence obtained by addition, deletion, or substitution of one or more nucleotides in a nucleotide sequence set forth in SEQ ID NO. 127.

The present disclosure also provides an isolated polynucleotide, encoding the guide RNA as described above.

The present disclosure also provides a construct, containing the isolated polynucleotide as described above.

The present disclosure also provides an expression system, containing the construct as described above or a genome exogenously introduced with the polynucleotide as described above.

The present disclosure also provides a gene editing system, including the guide RNA or the polynucleotide as described above.

The present disclosure also provides a composition, including the gene editing system, and a pharmaceutically acceptable carrier.

The present disclosure also provides a gene editing method, including contacting a target gene with the gene editing system as described above to realize edition of the target gene.

The present disclosure also provides a use of the guide RNA, the isolated polynucleotide, the construct, the expression system, the gene editing system, the pharmaceutical composition or the method as described above in gene editing of a target gene and/or a related polypeptide thereof in an *in vivo, ex vivo* cell or cell-free environment.

The present disclosure also provides a cell, including those that have been genetically modified with the AcC2C9 nuclease or a polynucleotide encoding the same, the guide RNA or a polynucleotide encoding the same, the recombinant expression vector, the system, or the composition as described above.

As described above, the guide RNA, the CRISPR/AcC2C9 gene editing system and the gene editing method of the present disclosure have the following beneficial effects:
(1) In the present disclosure, the guide RNA of AcC2C9 is thoroughly investigated, and the guide RNA with the highest editing efficiency is developed by performing several rounds of guide RNA modification, thus the CRISPR/AcC2C9 system which is an efficient and accurate genome editing tool is obtained. In the present disclosure, the editing efficiency of 35 target sequences across 6 endogenous genes is tested in a mammalian cell genome editing experiment, and the results show that the optimal version gRNA_M9 exhibits an enhanced gene editing efficiency across all the tested target sites. Compared with the original version gRNA_M1, the editing efficiency of 32 target sites of the optimized gRNA_M9 is about 2-10 times higher.
(2) The optimized version gRNA_M9 of the present disclosure enables the CRISPR/AcC2C9 system to exhibit editing activity at certain target sites where no activity is detected when using the original version gRNA_M1. Among the 35 tested target sites, there are 3 sites where no gene editing can be detected when using the original version gRNA_M1 as the guide RNA. However, when using the optimal version gRNA_M9, approximately 10% gene editing efficiency can be observed. This demonstrates that the guide RNA of the present disclosure improves the applicability of the small-sized CRISPR system in cellular gene editing.
(3) The guide RNA in the present disclosure has a significantly smaller size than conventional guide RNA, and after engineering the AcC2C9 system also has a small size, thus providing more possibilities for gene editing and gene therapy which are based on AAV transmission. This compact and small-sized CRISPR system has a broad application prospect in genome editing.

### BRIEF DESCRIPTION OF THE DRAWINGS

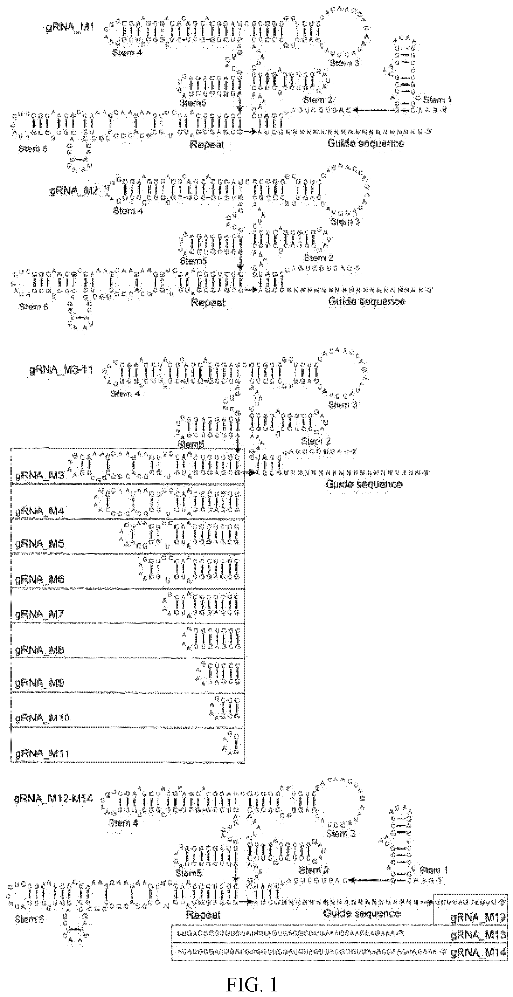

Fig. 1 is a schematic diagram showing the specific modification sites and corresponding base lengths for engineering of the original version guide RNA (gRNA_M1) and the optimized version guide RNA (M2-M14) in the present disclosure for the AcC2C9 nuclease in Example 1.

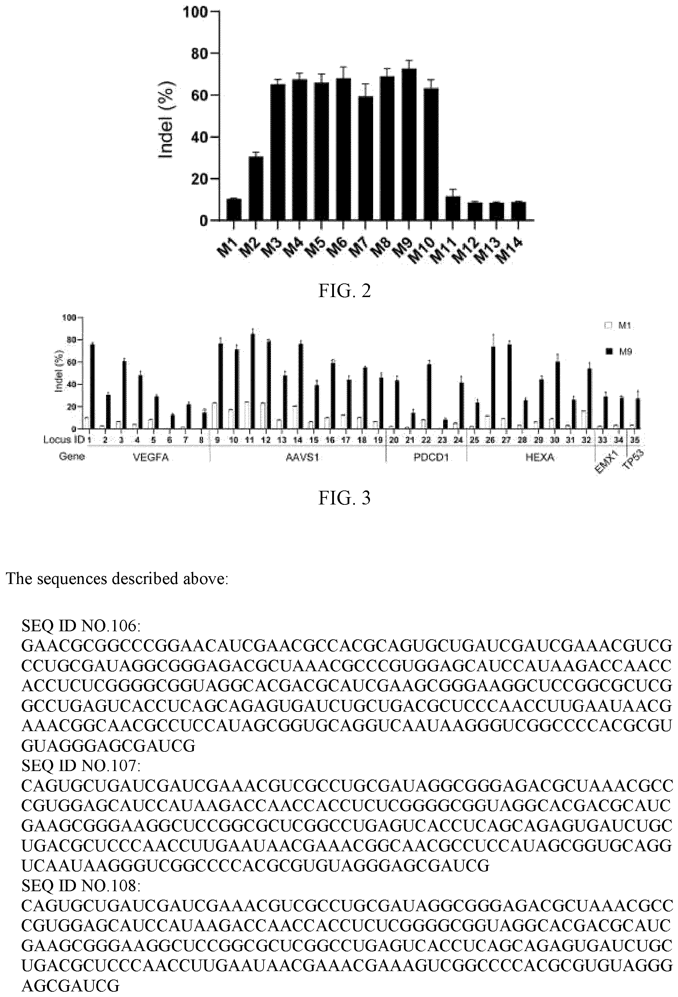

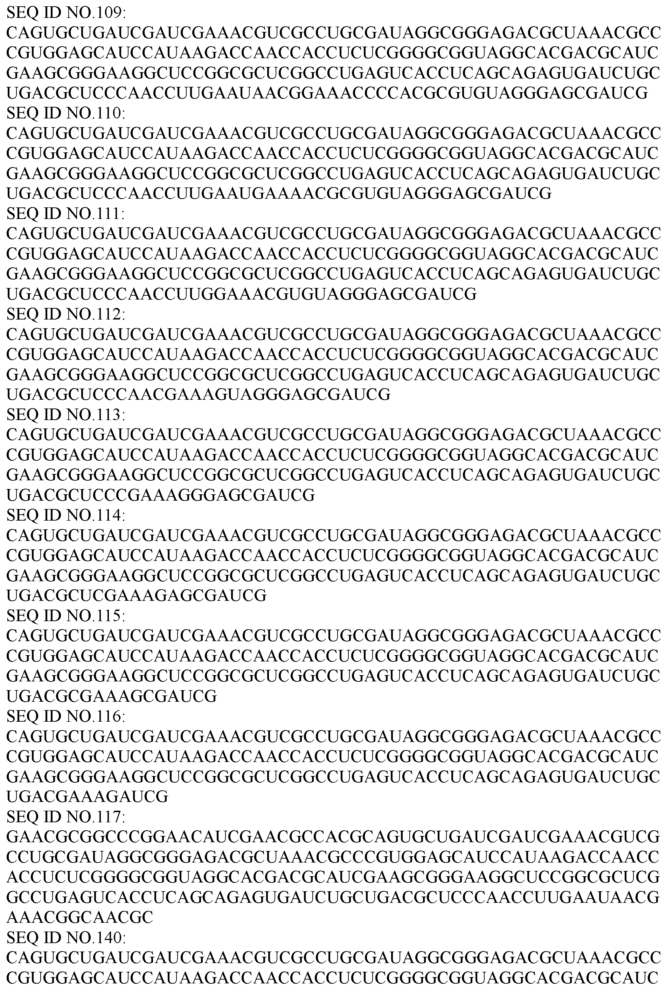

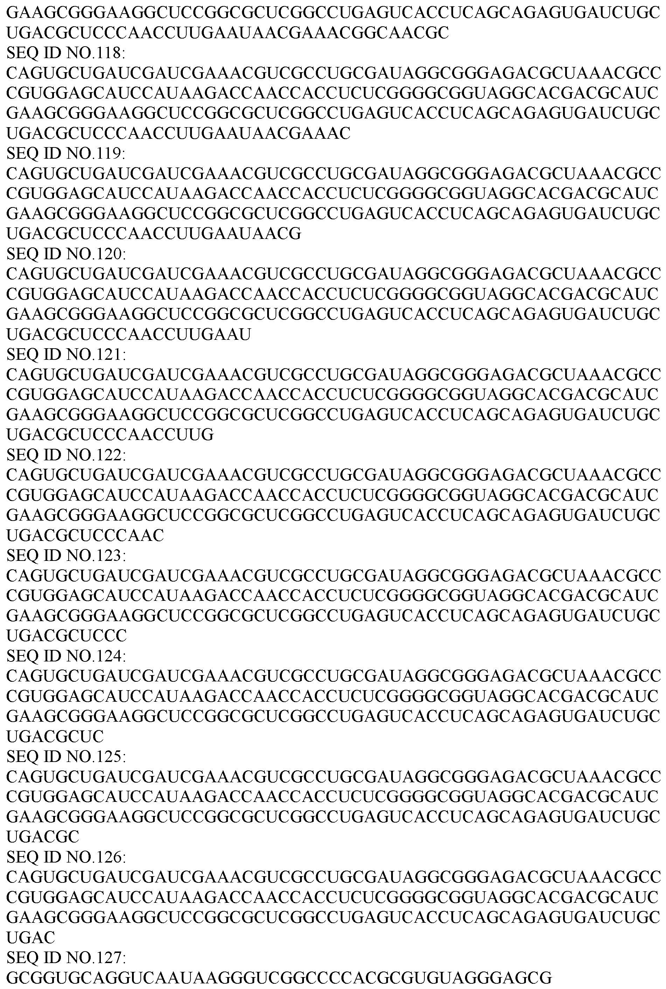

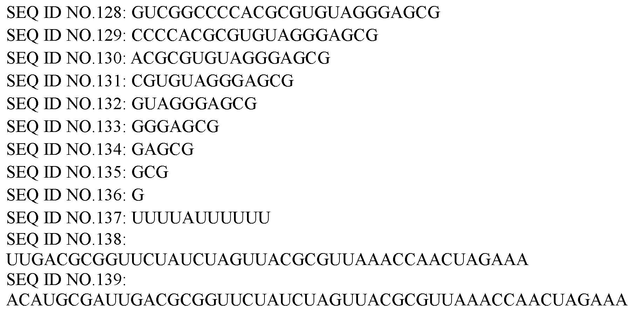

Fig. 2 is a diagram of the results of gene editing in mammalian cells mediated by the AcC2C9 nuclease with different versions of the guide RNA in Example 1. The results show that the editing efficiency of the partially engineered guide RNA is higher than that of the original version gRNA_M1, wherein gRNA_M9 has the highest efficiency.
Fig. 3 is a diagram of the results of gene editing in mammalian cells mediated by the AcC2C9 nuclease with the original version gRNA_M1, as well as the AcC2C9 nuclease with the optimal version gRNA_M9 in Example 2. The results show that the optimal version gRNA_M9 greatly improves the gene editing efficiency of a nuclease in mammalian cells; and the optimal version gRNA_M9 expands the gene editing range of a nuclease in mammalian cells.

### DETAILED DESCRIPTION OF THE PREFERRED EMBODIMENTS

The present disclosure provides a guide RNA (gRNA), comprising an RNA framework and a gene targeting section, wherein the nucleotide sequence of the RNA framework is shown as SEQ ID NOs. 106-107, or is a sequence obtained by addition, deletion, or substitution of one or more nucleotides in the nucleotide sequence set forth in SEQ ID NO. 106 or SEQ ID NO. 107.

The RNA framework includes a trans-activating CRISPR RNA (tracrRNA) and a tracr duplex. The tracrRNA and the tracr duplex can be linked directly or through a linker. The linker is composed of oligonucleotides; preferably, the number of oligonucleotides in the linker is 3-18 nt; further preferably, in one embodiment, the nucleotide sequence of the linker is GAAA.

In terms of the guide RNA described in the present disclosure, the tracr duplex and the tracrRNA sequence can be linked together to form a single RNA framework, i.e., the guide RNA is a single strand, which sequentially comprises the tracr RNA sequence and the tracr duplex sequence from the 5' end to the 3' end. When the guide RNA is one strand, the 3' end of the tracr RNA sequence and the 5' end of the tracr duplex sequence are linked directly or through a linker. When there is no linker, the tracrRNA sequence + the tracr duplex sequence forms the RNA framework; when there is a linker, the tracrRNA sequence + the linker + the tracr duplex sequence forms the RNA framework.

The guide RNA described in the present disclosure comprises sequences obtained by addition, deletion, or substitution of one or more nucleotides at the 5' or 3' end of the nucleotide sequence; e.g., sequences obtained by addition, deletion, or substitution of 15-150 nucleotides at the 5' or 3' end of the nucleotide sequence; or sequences with deletion of one or more nucleotides in the middle of the nucleotide sequence or sequences with substitution of 15-150 nucleotides in the middle of the nucleotide sequence, which are ligated using a linker, wherein the linker is composed of oligonucleotides. The number of the nucleotides being added, deleted or substituted can be 15-30, 30-45, 45-60, 60-75, 75-90, 90-105, 105-120, 120-135 or 135-150.

In the guide RNA described in the present disclosure, the gene targeting section is a nucleotide sequence complementary to the target sequence in the target gene. The gene targeting section is located at the 3' end of the RNA framework; and identifies the PAM sequence upstream/downstream of the target sequence; preferably, the PAM sequence is 5'-NAAN, wherein N is A, T, C, or G, and more preferably, the PAM sequence is 5'-NAAG. The gene targeting section targets the nucleic acid fragment with a length of 12-40 bp (for example, 13-20, 18-25, 22-32, 26-37, 30-38, or 32-40) downstream the PAM sequence, preferably, the gene targeting section targets the nucleic acid fragment with a length of 20 bp downstream the PAM sequence. In the present disclosure, the percentage of complementarity between the gene targeting section of the guide RNA and the target sequence in the target gene can be at least 50% (e.g., at least 55%, at least 60%, at least 65%, at least 70%, at least 75%, at least 80%, at least 85%, at least 90%, at least 95%, at least 97%, at least 98%, at least 99%, or 100%). In one embodiment, the nucleotide sequence of the gene targeting section is set forth in SEQ ID NO. 145.

The guide RNA described in the present disclosure comprises stem-loop structures that form a protein binding structure interacting with a nuclease such as AcC2C9. In some embodiments, the protein binding structure of the guide RNA comprises 6 stem-loop structures, including stem-loop 1, 2, 3, 4, 5 and 6 (as shown in Fig. 1, Stem1, Stem2, Stem3, Stem4, Stem5 and Stem6 respectively represent stem-loop 1-6). In some other embodiments, the protein binding structure of the guide RNA comprises 5 stem-loop structures, including stem-loop 2, 3, 4, 5 and 6 (i.e., the guide RNA with Stem1 in Fig. 1 being removed).

In one embodiment, the nucleotide sequence of the RNA framework is set forth in SEQ ID NO. 106, wherein the nucleotide sequence of the tracrRNA is set forth in SEQ ID NO. 117, and the nucleotide sequence of the tracr duplex is set forth in SEQ ID NO. 127. Furthermore, the nucleotide sequence of the guide RNA is set forth in SEQ ID NO. 4, and this guide RNA is referred to as gRNA_M1.

In one embodiment, the nucleotide sequence of the RNA framework is obtained by deletion of 30 bases in stem-loop 1 in the nucleotide sequence set forth in SEQ ID NO. 106. The nucleotide sequence of the RNA framework is set forth in SEQ ID NO. 107, wherein the nucleotide sequence of the tracrRNA is set forth in SEQ ID NO. 140, and the nucleotide sequence of the tracr duplex is set forth in SEQ ID NO. 127. Further, the nucleotide sequence of the guide RNA is set forth in SEQ ID NO. 5, and this guide RNA is referred to as gRNA_M2.

In one embodiment, the nucleotide sequence of the RNA framework is obtained by deletion of 34 bases in stem-loop 6 in the nucleotide sequence set forth in SEQ ID NO. 107. The nucleotide sequence of the RNA framework is set forth in SEQ ID NO. 108, wherein the nucleotide sequence of the tracrRNA is set forth in SEQ ID NO. 118, and the nucleotide sequence of the tracr duplex is set forth in SEQ ID NO. 128. Further, the nucleotide sequence of the guide RNA is set forth in SEQ ID NO. 6, and this guide RNA is referred to as gRNA_M3.

In one embodiment, the nucleotide sequence of the RNA framework is obtained by deletion of 43 bases in stem-loop 6 in the nucleotide sequence set forth in SEQ ID NO. 107, and the nucleotide sequence of the RNA framework is set forth in SEQ ID NO. 109, wherein the nucleotide sequence of the tracrRNA is set forth in SEQ ID NO. 119, and the nucleotide sequence of the tracr duplex is set forth in SEQ ID NO. 129. Further, the nucleotide sequence of the guide RNA is set forth in SEQ ID NO. 7, and this guide RNA is referred to as gRNA_M4.

In one embodiment, the nucleotide sequence of the RNA framework is obtained by deletion of 51 bases in stem-loop 6 in the nucleotide sequence set forth in SEQ ID NO. 107. The nucleotide sequence of the RNA framework is set forth in SEQ ID NO. 110, wherein the nucleotide sequence of the tracrRNA is set forth in SEQ ID NO. 120, and the nucleotide sequence of the tracr duplex is set forth in SEQ ID NO. 130. Further, the nucleotide sequence of the guide RNA is set forth in SEQ ID NO. 8, and this guide RNA is referred to as gRNA_M5.

In one embodiment, the nucleotide sequence of the RNA framework is obtained by deletion of 57 bases in stem-loop 6 in the nucleotide sequence set forth in SEQ ID NO. 107. The nucleotide sequence of the RNA framework is set forth in SEQ ID NO. 111, wherein the nucleotide sequence of the tracrRNA is set forth in SEQ ID NO. 121, and the nucleotide sequence of the tracr duplex is set forth in SEQ ID NO. 131. Further, the nucleotide sequence of the guide RNA is set forth in SEQ ID NO. 9, and this guide RNA is referred to as gRNA_M6.

In one embodiment, the nucleotide sequence of the RNA framework is obtained by deletion of 64 bases in stem-loop 6 in the nucleotide sequence set forth in SEQ ID NO. 107. The nucleotide sequence of the RNA framework is set forth in SEQ ID NO. 112, wherein the nucleotide sequence of the tracrRNA is set forth in SEQ ID NO. 122, and the nucleotide sequence of the tracr duplex is set forth in SEQ ID NO. 132. Further, the nucleotide sequence of the guide RNA is set forth in SEQ ID NO. 10, and this guide RNA is referred to as gRNA_M7.

In one embodiment, the nucleotide sequence of the RNA framework is obtained by deletion of 70 bases in stem-loop 6 in the nucleotide sequence set forth in SEQ ID NO. 107. The nucleotide sequence of the RNA framework is set forth in SEQ ID NO. 113, wherein the nucleotide sequence of the tracrRNA is set forth in SEQ ID NO. 123, and the nucleotide sequence of the tracr duplex is set forth in SEQ ID NO. 133. Further, the nucleotide sequence of the guide RNA is set forth in SEQ ID NO. 11, and this guide RNA is referred to as gRNA_M8.

In one embodiment, the nucleotide sequence of the RNA framework is obtained by deletion of 74 bases in stem-loop 6 in the nucleotide sequence set forth in SEQ ID NO. 107. The nucleotide sequence of the RNA framework is set forth in SEQ ID NO. 114, wherein the nucleotide sequence of the tracrRNA is set forth in SEQ ID NO. 124, and the nucleotide sequence of the tracr duplex is set forth in SEQ ID NO. 134. Further, the nucleotide sequence of the guide RNA is set forth in SEQ ID NO. 12, and this guide RNA is referred to as gRNA_M9.

In one embodiment, the nucleotide sequence of the RNA framework is obtained by deletion of 78 bases in stem-loop 6 in the nucleotide sequence set forth in SEQ ID NO. 107. The nucleotide sequence of the RNA framework is set forth in SEQ ID NO. 115, wherein the nucleotide sequence of the tracrRNA is set forth in SEQ ID NO. 125, and the nucleotide sequence of the tracr duplex is set forth in SEQ ID NO. 135. Further, the nucleotide sequence of the guide RNA is set forth in SEQ ID NO. 13, and this guide RNA is referred to as gRNA_M10.

In one embodiment, the nucleotide sequence of the RNA framework is obtained by deletion of 82 bases in stem-loop 6 in the nucleotide sequence set forth in SEQ ID NO. 107. The nucleotide sequence of the RNA framework is set forth in SEQ ID NO. 116, wherein the nucleotide sequence of the tracrRNA is set forth in SEQ ID NO. 126, and the nucleotide sequence of the tracr duplex is set forth in SEQ ID NO. 136. Further, the nucleotide sequence of the guide RNA is set forth in SEQ ID NO. 14, and this guide RNA is referred to as gRNA_M11.

In some embodiments of the present disclosure, the guide RNA further includes an RNA structure stabilizing sequence provided at the 3' end of the guide RNA.

In some embodiments of the present disclosure, the RNA structure stabilizing sequence is set forth in SEQ ID NOs. 137-139.

In one embodiment, the RNA structure stabilizing sequence TTTTATTTTTT is provided adjacent to the gene targeting section of gRNA_M1 to obtain gRNA_M12. Further, the nucleotide sequence of the gRNA_M12 is set forth in SEQ ID NO. 15.

In one embodiment, the RNA structure stabilizing sequence TTGACGCGGTTCTATCTAGTTACGCGTTAAACCAACTAGAAA is provided adjacent to the gene targeting section of gRNA_M1 to obtain gRNA_M13. The nucleotide sequence of the gRNA_M13 is set forth in SEQ ID NO. 16.

In one embodiment, the RNA structure stabilizing sequence ACATGCGATTGACGCGGTTCTATCTAGTTACGCGTTAAACCAACTAGAAA is provided adjacent to the gene targeting section of gRNA_M1 to obtain gRNA_M14. The nucleotide sequence of the gRNA_M14 is set forth in SEQ ID NO. 17.

In some embodiments, the guide RNA further comprises a transcription terminator.

In the guide RNA described in the present disclosure, the gene targeting section comprises a nucleotide sequence complementary to a target sequence in a target gene. The target sequence of the target gene hybridizes with (i.e., base pairing) the gene targeting section in a sequence-specific manner. The gene targeting section of the guide RNA can be modified by, for example, genetic engineering, so that the guide RNA hybridizes with any desired sequence within the target gene. The guide RNA guides the bound polypeptide to a specific nucleotide sequence in the target gene through the gene targeting section.

In some embodiments, the target gene is a DNA sequence. In some embodiments, the target gene is an RNA sequence.

The present disclosure further provides a method for modification of the guide RNA. The variant sequence of the guide RNA refers to a sequence obtained by addition, deletion, or substitution of one or more nucleotides in the nucleotide sequences set forth in SEQ ID NO. 4-SEQ ID NO. 5. Preferably, the variant sequence of the guide RNA refers to a sequence obtained by deletion of nucleotides at the 5' end and/or 3' end of the nucleotide sequences set forth in SEQ ID NO. 4-SEQ ID NO. 5, i.e., a certain number of nucleotides can be deleted or truncated at the 5' end only, a certain number of nucleotides can be deleted or truncated at the 3' end only, or a certain number of nucleotides can be deleted or truncated at the 5' end and the 3' end simultaneously. Preferably, the variant sequence of the guide RNA refers to a sequence obtained by truncation, addition or substitution of nucleotides within the nucleotide sequences set forth in SEQ ID NO. 4-SEQ ID NO. 5 (other than 5' end and/or 3' end), i.e., any combination of truncation, addition and substitution of nucleotides at any one or more positions in the middle of the nucleotide sequence. Preferably, the variant sequence of the guide RNA refers to a sequence obtained by addition, deletion, or substitution of one or more nucleotides at the 5' end and/or the 3' end and/or any position in the middle of the nucleotide sequences set forth in SEQ ID NOs. 4-5.

In one embodiment, the variant sequence of the guide RNA is obtained by deletion of 30 bases in stem-loop 1 in the nucleotide sequence set forth in SEQ ID NO. 4, and the nucleotide obtained by the deletion is referred to as gRNA_M2; the nucleotide sequence of gRNA_M2 is set forth in SEQ ID NO. 5.

In one embodiment, the variant sequence of the guide RNA is obtained by deletion of 34 nt bases in stem-loop 6 in the nucleotide sequence set forth in SEQ ID NO. 5, and a GAAA linker is added at the truncated position to obtain gRNA_M3; the nucleotide sequence of gRNA_M3 is set forth in SEQ ID NO. 6.

In one embodiment, the variant sequence of the guide RNA is obtained by deletion of 43 nt bases in stem-loop 6 in the nucleotide sequence set forth in SEQ ID NO. 5, and a GAAA linker is added at the truncated position to obtain gRNA_M4; the nucleotide sequence of gRNA_M4 is set forth in SEQ ID NO. 7.

In one embodiment, the variant sequence of the guide RNA is obtained by deletion of 51 nt bases in stem-loop 6 in the nucleotide sequence set forth in SEQ ID NO. 5, and a GAAA linker is added at the truncated position to obtain gRNA_M5; the nucleotide sequence of gRNA_M5 is set forth in SEQ ID NO. 8.

In one embodiment, the variant sequence of the guide RNA is obtained by deletion of 57 nt bases in stem-loop 6 in the nucleotide sequence set forth in SEQ ID NO. 5, and a GAAA linker is added at the truncated position to obtain gRNA_M6; the nucleotide sequence of gRNA_M6 is set forth in SEQ ID NO. 9.

In one embodiment, the variant sequence of the guide RNA is obtained by deletion of 64 nt bases in stem-loop 6 in the nucleotide sequence set forth in SEQ ID NO. 5, and a GAAA linker is added at the truncated position to obtain gRNA_M7; the nucleotide sequence of gRNA_M7 is set forth in SEQ ID NO. 10.

In one embodiment, the variant sequence of the guide RNA is obtained by deletion of 70 nt bases in stem-loop 6 in the nucleotide sequence set forth in SEQ ID NO. 5, and a GAAA linker is added at the truncated position to obtain gRNA_M8; the nucleotide sequence of gRNA_M8 is set forth in SEQ ID NO. 11.

In one embodiment, the variant sequence of the guide RNA is obtained by deletion of 74 nt bases in stem-loop 6 in the nucleotide sequence set forth in SEQ ID NO. 5, and a GAAA linker is added at the truncated position to obtain gRNA_M9; the nucleotide sequence of gRNA_M9 is set forth in SEQ ID NO. 12.

In one embodiment, the variant sequence of the guide RNA is obtained by deletion of 78 nt bases in stem-loop 6 in the nucleotide sequence set forth in SEQ ID NO. 5, and a GAAA linker is added at the truncated position to obtain gRNA_M10; the nucleotide sequence of gRNA_M10 is set forth in SEQ ID NO. 13.

In one embodiment, the variant sequence of the guide RNA is obtained by deletion of 82 nt bases in stem-loop 6 in the nucleotide sequence set forth in SEQ ID NO. 5, and a GAAA linker is added at the truncated position to obtain gRNA_M11; the nucleotide sequence of gRNA_M11 is set forth in SEQ ID NO. 14.

In one embodiment, the variant sequence of the guide RNA is obtained by addition of TTTTATTTTTT at the 3' end of the nucleotide sequence set forth in SEQ ID NO. 4 and this variant sequence of the guide RNA is referred to asgRNA_M12; the nucleotide sequence of gRNA_M12 is set forth in SEQ ID NO. 15.

In one embodiment, the variant sequence of the guide RNA is obtained by addition of TTGACGCGGTTCTATCTAGTTACGCGTTAAACCAACTAGAAA at the 3' end of the nucleotide sequence set forth in SEQ ID NO. 4 and this variant sequence of the guide RNA is referred to as gRNA_M13; the nucleotide sequence of gRNA_M13 is set forth in SEQ ID NO. 16.

In one embodiment, the variant sequence of the guide RNA is obtained by addition of
ACATGCGATTGACGCGGTTCTATCTAGTTACGCGTTAAACCAACTAGAAA at the 3' end of the nucleotide sequence set forth in SEQ ID NO. 4 and this variant sequence of the guide RNA is referred to as gRNA_M14; the nucleotide sequence of gRNA_M14 is set forth in SEQ ID NO. 17.

The present disclosure also provides a modified guide RNA, which can achieve hybridization with any desired sequence in the target gene through modification; or, the properties of which can be altered through modification, such as enhancing the stability of the guide RNA through modification, including but not limited to prolonging its half-life in cells by increasing its resistance to degradation caused by ribonucleases (RNases) in cells; or, which can enhance the formation or stability of the CRISPR-AcC2C9 genome editing complex comprising the guide RNA and an endonuclease (e.g., the AcC2C9 nuclease) through modification; or, which can enhance the specificity of the genome editing complex through modification; or, which can enhance the initiation site, stability, or kinetics of the interaction between the genome editing complex and the target sequence in the genome through modification; or, which can reduce the likelihood or extent of innate immune responses triggered by the RNA introduced into the cell through modification. In the present disclosure, multiple properties of the CRISPR-AcC2C9 system (as described below) can be altered through modifications to the guide RNA, such as enhancing the formation, on-target activity, specificity, stability, or kinetic characteristics of the CRISPR-AcC2C9 genome editing complex. RNA can be modified using known methods in the art, including but not limited to 2'-fluoro, 2'-amino modifications, etc. on the ribose of pyrimidine, base residues, or the inverted bases at the 3' end of RNA. In the present disclosure, the guide RNA can undergo any one modification or a combination of multiple modifications. In some embodiments, the guide RNA introduced into the cell is modified to edit the loci of any one or more genomes.

The present disclosure also provides an isolated polynucleotide, encoding any of the guide RNA as described above.

The present disclosure also provides a construct, containing the isolated polynucleotide as described above. The construct can typically be obtained by inserting the isolated polynucleotide into a suitable expression vector for construction, and a skilled person in the art can select a suitable expression vector. The construct, for example, can be a recombinant expression vector, and any suitable expression vector can be used as long as it is compatible with the host cell, including but not limited to viral vectors (e.g., poxvirus-based viral vectors; poliovirus; adenovirus; adeno-associated virus; SV40; herpes simplex virus; human immunodeficiency virus); retroviral vectors (e.g., murine leukemia virus, spleen necrosis virus, and vectors derived from retroviruses, e.g., Rous sarcoma virus, Harvey sarcoma virus, avian leukosis virus, lentivirus, human immunodeficiency virus, myeloproliferative sarcoma virus, and mammary tumor virus), etc.

In certain embodiments, multiple guide RNAs are used simultaneously in the same cell to regulate transcription at different positions on the same target gene or different target genes. When multiple guide RNAs are used simultaneously, they can exist on the same expression vector or different vectors, and can also be expressed simultaneously; when they exist on the same vector, they can be expressed under the same control element.

In certain embodiments, the nucleotide sequence encoding the guide RNA is operably linked to a control element, e.g., a transcription control element, for example, a promoter. In certain embodiments, the nucleotide sequence encoding the guide RNA is operably linked to an inducible promoter. In certain embodiments, the nucleotide sequence encoding the guide RNA is operably linked to a constitutive promoter. A transcription control element can function in eukaryotic cells, e.g., mammalian cells (HEK293T cells); or prokaryotic cells (e.g., bacterial or archaeal cells). In certain embodiments, the nucleotide sequence encoding the guide RNA is operably linked to multiple control elements, allowing the expression of the nucleotide sequence encoding the guide RNA in both prokaryotic and eukaryotic cells.

In the present disclosure, the guide RNA can be synthesized by artificial synthesis methods, for example, by chemical methods, so that it can be easily modified in multiple ways. Any modification method known in the art can be used, for example, polyA tailing, addition of a 5' cap analogue, inclusion of a 5' or 3' untranslated region (UTR), modification of the 5' or 3' end with thiophosphorylated 2'-O-methyl base, or phosphatase treatment to remove 5' end phosphate ester, etc.

In some embodiments, the nucleotide sequence encoding the guide RNA comprises one or more modifications, which can, for example, enhance activity, stability or specificity, alter delivery, reduce the innate immune response in host cells or for other enhancements.

In some embodiments, one or more targeting moieties or conjugates that enhance the activity, cell distribution or cell uptake of the nucleotide sequence encoding the guide RNA are chemically linked to the guide RNA. The targeting moiety or conjugate may include a conjugate group covalently bound to a functional group; the conjugate group includes reporter molecules, polyamines and polyethylene glycol. In some embodiments, groups that enhance pharmacodynamic properties are linked to gRNA, and the groups include those improve uptake, enhance resistance to degradation, and/or enhance sequence-specific hybridization with a target nucleic acid.

In the present disclosure, the nucleic acid comprising the polynucleotide encoding the guide RNA may be a nucleic acid mimics. For example, polynucleotide mimics peptide nucleic acids with good hybridization properties, etc.

In the present disclosure, the guide RNA or the polynucleotide encoding the guide RNA is suitable for any biological or in-vitro environment, including but not limited to bacteria, archaea, fungi, protozoa, plants and animals. Accordingly, suitable target cells include, but are not limited to, bacterial cells, archaea cells, fungal cells, protozoan cells, plant cells and animal cells. Suitable target cells can be any type of cell, including stem cells, somatic cells, etc.

The present disclosure also provides an expression system, containing the construct as described above or a genome exogenously introducing the polynucleotide as described above. The host cell of the expression system is selected from eukaryotic cells and prokaryotic cells; preferably, the host cell is selected from mouse cells and human cells.

The present disclosure also provides a gene editing system, including the guide RNA or the polynucleotide as described above; further, it may also include a nuclease or a polynucleotide encoding the same.

In the editing system described in the present disclosure, the polynucleotide encoding the nuclease includes: an encoding sequence encoding the nuclease only; an encoding sequence of the nuclease and various additional encoding sequences; an encoding sequence of the nuclease (and any additional encoding sequences) and a non-encoding sequence. The polynucleotide encoding the guide RNA includes: an encoding sequence encoding the guide RNA only; an encoding sequence of the guide RNA and various additional encoding sequences; an encoding sequence of the guide RNA (and any additional encoding sequences) and a non-encoding sequence. In some embodiments, the gene editing system comprises one or more vectors; the one or more vectors comprise (i) a first regulatory element operably linked to the polynucleotide encoding the nuclease; and (ii) a second regulatory element operably linked to the polynucleotide encoding the guide RNA nucleotide sequence; the (i) and (ii) are on the same vector or different vectors. In some embodiments, the gene editing system comprises (i) a nuclease or a variant thereof, and (ii) a vector comprising the sequence encoding the guide RNA. In another embodiment, the system comprises a complex of the guide RNA and the nuclease.

The first regulatory element can regulate the transcription of the polynucleotide encoding the nuclease or its variant. The polynucleotide encoding the nuclease or its variants may be one or more, and the first regulatory element may be one or more. The second regulatory element can regulate the transcription of the polynucleotide encoding the guide RNA. The polynucleotide encoding the guide RNA may be one or more, and the second regulatory element may be one or more.

The system described in the present disclosure can comprise one guide RNA or comprise multiple guide RNAs simultaneously. In one embodiment, the system comprises multiple guide RNAs to modify different positions on the same target DNA or different target DNAs simultaneously. In one embodiment, two or more guide RNAs target the same gene or transcript or loci. In one embodiment, two or more guide RNAs target different unrelated locus. In some embodiments, two or more guide RNAs target different but related locus.

In the gene editing system described in the present disclosure, the nuclease is a CRISPR nuclease; preferably, the nuclease is selected from Cas9, Cas12, Cas13 protein families and variants thereof; further preferably, the Cas nuclease is selected from nSpCas9 and a variant thereof, SaCas9 and a variant thereof, Cas12a and a variant thereof, as well as C2C9 and a variant thereof; more preferably, the Cas nuclease is an AcC2C9 nuclease or a variant thereof. In some embodiments, the AcC2C9 nuclease is directly provided as a protein; for example, acting as an exogenous protein to transform spheroplast and/or using nucleic acid of the AcC2C9 nuclease to transform fungi. The AcC2C9 nuclease can be introduced into a cell by any suitable method, such as injection and the like. The gene editing system described in the present disclosure identifies the PAM sequence upstream of the target sequence; preferably, the PAM sequence is 5'-NAAN, wherein N is A, T, C, or G; and more preferably, the PAM sequence is 5'-NAAG. The gene editing system targets a nucleic acid fragment with a length of 12-40 bp (such as 20 bp) downstream of the PAM sequence. The gene editing system targets at least one target sequence in the cell genome.

In certain embodiments, the nucleic acid encoding the AcC2C9 nuclease is DNA. In certain embodiments, the nucleic acid encoding the AcC2C9 nuclease is RNA. In certain embodiments, the nucleic acid encoding the AcC2C9 nuclease is an expression vector, e.g., a recombinant expression vector. Any suitable expression vector can be used as long as it is compatible with the host cell, including but not limited to viral vectors (e.g., poxvirus-based viral vectors; poliovirus; adenovirus; adeno-associated virus; SV40; herpes simplex virus; human immunodeficiency virus); retroviral vectors (e.g., murine leukemia virus, spleen necrosis virus, and vectors derived from retroviruses, e.g., Rous sarcoma virus, Harvey sarcoma virus, avian leukosis virus, lentivirus, human immunodeficiency virus, myeloproliferative sarcoma virus, and mammary tumor virus), etc.

In one embodiment, the nucleic acid encoding the AcC2C9 nuclease is set forth in SEQ ID NO. 2. In one embodiment, the present disclosure provides a codon-optimized polynucleotide sequence of the AcC2C9 nuclease, which has at least 90%, 92%, 93%, 94%, 95%, 96%, 97%, 98%, 99%, 99.2%, 99.5%, 99.8%, 99.9%, or 100% sequence homology to SEQ ID NO: 2. In a preferred embodiment, the human codon-optimized sequence encoding the AcC2C9 nuclease is set forth in SEQ ID NO. 3, which encodes one or more functional AcC2C9 domains or a polypeptide having the same function as the polypeptide encoded by the original natural nucleotide sequence.

In certain embodiments, the nucleotide sequence encoding the AcC2C9 nuclease is operably linked to a control element, e.g., a transcription control element, for example, a promoter. In certain embodiments, the nucleotide sequence encoding the AcC2C9 nuclease is operably linked to an inducible promoter. In certain embodiments, the nucleotide sequence encoding the AcC2C9 nuclease is operably linked to a constitutive promoter. A transcription control element can function in eukaryotic cells, e.g., mammalian cells (HEK293T cells); or prokaryotic cells (e.g., bacterial or archaeal cells). In certain embodiments, the nucleotide sequence encoding the AcC2C9 nuclease is operably linked to multiple control elements, allowing the expression of the nucleotide sequence encoding the AcC2C9 nuclease in both prokaryotic and eukaryotic cells. In some embodiments, the polynucleotide sequence encoding the AcC2C9 nuclease is operably linked to a suitable nuclear localization signal for expression in a cellular or in-vitro environment.

In the present disclosure, the polynucleotide encoding the AcC2C9 nuclease can be synthesized by artificial synthesis methods, for example, by chemical methods, so that it can be easily modified in multiple ways. Any modification method known in the art can be used. In some embodiments, the polynucleotide encoding the AcC2C9 nuclease comprises one or more modifications, e.g., modifications for enhancing transcription activity, changing enzyme activity, improving its translation or stability (e.g., increasing its resistance to proteolysis and degradation) or specificity, changing solubility, changing delivery, and reducing innate immune response in host cells. The modification can use any modification method known in the art. In some embodiments, the DNA or RNA encoding the AcC2C9 nuclease introduced into the cell is modified to edit the loci of any one or more genomes. In some embodiments, the nucleic acid sequence encoding the AcC2C9 nuclease is a modified nucleic acid, e.g., a codon-optimized nucleic acid. The modification may be a single modification or a combination modification.

In the present disclosure, the nucleic acid comprising the polynucleotide encoding the AcC2C9 nuclease may be a nucleic acid mimics. For example, polynucleotide mimics peptide nucleic acids with good hybridization properties, etc.

In the present disclosure, the AcC2C9 nuclease or the polynucleotide encoding the AcC2C9 nuclease is suitable for any biological or in-vitro environment, including but not limited to bacteria, archaea, fungi, protozoa, plants or animals. Accordingly, suitable target cells include, but are not limited to, eukaryotic cells and prokaryotic cells, e.g., bacterial cells, archaea cells, fungal cells, protozoan cells, plant cells or animal cells; the eukaryotic cells comprise mammalian cells and plant cells, and the prokaryotic cells include *E*. *coli* and *Klebsiella pneumoniae.* Suitable target cells can be any type of cell, including stem cells, somatic cells, etc. The present disclosure is preferably used for the mammalian cell HEK293T cell. The cells can be *in vivo* or *ex vivo.* In some embodiments, the AcC2C9 nuclease or the nucleic acid encoding the AcC2C9 nuclease is formulated in liposomes or lipid nanoparticles.

In the system described in the present disclosure, the AcC2C9 nuclease and the guide RNA can form a complex in a host cell, and recognize a PAM sequence upstream of a target gene (such as a target DNA) sequence; the target sequence of the CRISPR/AcC2C9 gene editing system is a nucleic acid fragment (such as a DNA fragment) with a length of 20 bp downstream the PAM sequence. In one embodiment, the complex can selectively regulate the transcription of a target DNA in a host cell. The CRISPR/AcC2C9 gene editing system can cleave the double strand of the target DNA, resulting in DNA breakage.

In one embodiment, the system comprises a recombinant expression vector. In one embodiment, the system comprises a recombinant expression vector comprising (i) a nucleotide sequence encoding a guide RNA, wherein the guide RNA comprises: (a) a first section comprising a nucleotide sequence complementary to a sequence in a target DNA; and (b) a second section interacting with the AcC2C9 nuclease; and (ii) a nucleotide sequence encoding the AcC2C9 nuclease, wherein the AcC2C9 nuclease comprises: (a) an RNA-binding domain which interacts with the guide RNA; and (b) a domain which regulates transcription of the target DNA, wherein the site for regulating transcription in the target DNA is determined by the guide RNA.

In the present disclosure, an AcC2C9 nuclease variant can also be formed through modification, mutation, DNA shuffling, etc., so that the AcC2C9 nuclease variant has improved and desired characteristics, e.g., function, activity, kinetics, half-life, etc. The modification can be, for example, deletion, insertion or substitution of amino acids, and can also be, for example, replacement of the "cleavage domain" of AcC2C9 nucleases with homologous or heterologous cleavage domains from other different nucleases (e.g., the HNH domain of a CRISPR-related nuclease); proteins can be modified by any method based on DNA binding and/or DNA modifying known in the art, such as methylation, demethylation, acetylation, etc., so that for example, the DNA targeting of the AcC2C9 nuclease can be altered. The DNA shuffling refers to exchanging sequence fragments between DNA sequences of AcC2C9 nucleases from different sources, to generate chimeric DNA sequences encoding synthetic proteins with RNA-directed endonuclease activity. The modification, mutation, DNA shuffling, etc. can be used singly or in combination.

Specifically, the AcC2C9 nuclease described in the present disclosure can be:
(I) a wild-type AcC2C9 nuclease or a fragment thereof which is directed by RNA and has a nucleic acid binding activity towards RNA; the AcC2C9 nuclease is derived from *Actinomadura craniellae* C2C9, with an amino acid sequence set forth in SEQ ID NO. 1; preferably, a humanized codon-optimized nucleic acid sequence of the AcC2C9 nuclease is set forth in SEQ ID NO. 3;
(II) a variant having at least 50% sequence homology with the amino acid sequence in (I) and having a nucleic acid binding activity towards RNA and being directed by RNA;
(III) based on (I) or (II), the AcC2C9 nuclease further includes a nuclear localization signal fragment;
(IV) based on (I) or (II) or (III), the AcC2C9 nuclease further comprises:
   (a) one or more modifications or mutations, which result in a significantly reduced endonuclease activity or a loss of endonuclease activity; and/or
   (b) polypeptides or domains with other functional activities; and
(V) based on (I) or (II) or (III), the AcC2C9 nuclease has an endonuclease activity.

In some embodiments, the AcC2C9 can be used in combination with other enzymes or other components to further develop various potential applications of the AcC2C9 nuclease. Non-limiting examples of the AcC2C9 nuclease variant in (IV) include for example, a single-gene editing system based on the AcC2C9 nuclease by fusing inactivated AcC2C9 with a base deaminase; a Prime editing system based on the AcC2C9 nuclease by fusing inactivated AcC2C9 with a reverse transcriptase; a transcription activation system based on the AcC2C9 nuclease by fusing inactivated AcC2C9 with a transcription activation factor; an epigenetic modification system based on the AcC2C9 nuclease by fusing inactivated AcC2C9 with a nucleic acid epigenetic modifying enzyme; and a transcription repression system based on the AcC2C9 nuclease by utilizing inactivated AcC2C9.

Specific properties of the AcC2C9 nuclease variant are as follows, including but not limited to:
having enhanced or reduced ability to bind to target sites, or retaining ability to bind to target sites;
having enhanced or reduced endoribonuclease and/or endonuclease activity, or retaining endoribonuclease and/or endonuclease activity;
having deaminase activity, which can act on cytosine, guanine, or adenine bases, followed by the deaminated site replication and repair within a cell, generating guanine, thymine, and guanine, respectively;
having ability to regulate transcription of a target DNA, either increasing or decreasing transcription of the target DNA at specific positions;
altering DNA targeting;
having increased, decreased, or maintained stability;
having ability to cleave the complementary strand of a target DNA, but having reduced ability to cleave the non-complementary strand;
having ability to cleave the non-complementary strand of a target DNA, but having reduced ability to cleave the complementary strand;
having reduced ability to cleave both the complementary and non-complementary strands of a target DNA;
having enzymatic activity to modify polypeptides (e.g., histone) associated with DNA, wherein the enzymatic activity can be one or more of methyltransferase activity, demethylase activity, acetyltransferase activity, deacetylase activity, kinase activity, phosphatase activity, ubiquitin ligase activity, deubiquitinating activity, ribosylation activity, etc. (covalent modification of proteins is catalyzed by these enzyme activities; for example, the AcC2C9 nuclease variants modify histone through methylation, acetylation, ubiquitination, phosphorylation, etc., to induce structural changes in histone-associated DNA, thereby controlling the structure and properties of DNA).

In some embodiments, the AcC2C9 nuclease variant has no cleavage activity. In some embodiments, the AcC2C9 nuclease variant has single-strand cleavage activity. In some embodiments, the AcC2C9 nuclease variant has double-strand cleavage activity.

Having enhanced activity or ability refers to the activity or ability being improved by at least 1%, 5%, 10%, 20%, 30%, 40% or 50% relative to the wild-type AcC2C9 nuclease.

Having reduced activity and ability refers to the activity or ability is less than 50%, 40% lower than, 30% lower than, 20% lower than, 10% lower than, 5% lower than or 1% lower than that of the wild-type AcC2C9 nuclease.

These small-sized AcC2C9 and variants thereof described in the present disclosure can be used in any system, composition, kit and method described in the present disclosure below.

Unless otherwise specified, the terms "AcC2C9" and " AcC2C9 nuclease" include the wild-type AcC2C9 nuclease and all variants thereof, and those skilled in the art can determine the types of AcC2C9 nuclease variants by conventional methods which are not limited to those listed above.

Each component in the system described in the present disclosure can be transported by carriers. For example, for polynucleotides, methods that can be used include, but are not limited to, nanoparticles, liposomes, ribonucleoprotein, small molecule RNA-conjugates, chimeras, RNA-fusion protein complexes, etc.

The system described in the present disclosure may further include one or more donor templates. In certain embodiments, the donor template comprises a donor sequence for inserting into a target gene.

The system described in the present disclosure may further comprise a dimeric FOK1 nuclease, and an intact or partially or completely defective AcC2C9 nuclease or a guide RNA is linked to the dimeric FOK1 nuclease to direct endonuclease cleavage after being guided to one or more specific DNA target sites using one or more guide RNA molecules.

The system described in the present disclosure can edit or modify DNA at multiple positions in cells for gene therapy, including but not limited to gene therapy for diseases, biological research, crop resistance improvement or yield improvement, etc.

The present disclosure also provides a composition, that comprises one or more of the AcC2C9 nuclease or a polynucleotide encoding the same, the guide RNA or a polynucleotide encoding the same, the recombinant expression vector, and the system as described above, and acceptable carriers, vehicles, etc. The acceptable carriers and vehicles are for example sterile water or normal saline, stabilizers, excipients, antioxidants (ascorbic acid, etc.), buffers (phosphoric acid, citric acid, other organic acids, etc.), preservatives, surfactants (PEG, Tween, etc.), chelating agents (EDTA, etc.), adhesives, etc. In addition, the composition can also contain other low molecular weight polypeptides; proteins such as serum albumin, gelatin or immunoglobulins; amino acids such as glycine, glutamine, asparagine, arginine and lysine; saccharides (such as polysaccharides and monosaccharides) or carbohydrates; sugar alcohols such as mannitol or sorbitol. When preparing aqueous solution for injection, e.g., normal saline, an isotonic solution containing glucose or other auxiliary drugs, such as D-sorbitol, D-mannose, D-mannitol and sodium chloride, appropriate solubilizers for example, alcohols (ethanol, etc.), polyols (propylene glycol, PEG, etc.) and nonionic surfactants (Tween 80 or HCO-50) and the like can be used. In some embodiments, the composition comprises a guide RNA and a buffer for stabilizing nucleic acids.

The present disclosure also provides a kit, including the system or composition as described above. The kit may further include one or more of, for example, a dilution buffer; a washing buffer; a control reagent, etc. In some embodiments, the kit includes (a) the AcC2C9 nuclease as described above or a nucleic acid encoding the AcC2C9 nuclease; and (b) a guide RNA or a nucleic acid encoding the guide RNA, wherein the guide RNA can direct the AcC2C9 nuclease or a variant thereof to a target polynucleotide sequence. In certain embodiments, the kit further contains a donor template comprising a heterologous polynucleotide sequence, wherein the heterologous polynucleotide sequence can be inserted into the target polynucleotide sequence.

The present disclosure also provides a gene editing method, including contacting a target gene with the gene editing system as described above to realize editing of the target gene. The method of the present disclosure can be used to target, edit, modify or manipulate a target gene (such as a target DNA) in cells, in *in vivo,* in *ex vivo* cell, or in cell-free system, and the method includes: introducing the AcC2C9 nuclease or a polynucleotide encoding the same, the guide RNA or a polynucleotide encoding the same, the recombinant expression vector, the system, the composition, etc., into a kit to target, edit, modify or manipulate the target gene in *in vivo, ex vivo* cell or cell-free system. In one embodiment, the method includes the following steps:
(a) introducing the AcC2C9 nuclease or the nucleic acid encoding the AcC2C9 nuclease in an *in vivo, ex vivo* cell or cell-free system;
(b) introducing the guide RNA (gRNA) or the nucleic acid (e.g., DNA) suitable for in situ generation of such guide RNA; and
(c) contacting cells or target genes with the AcC2C9 nuclease or the nucleic acid encoding the AcC2C9 nuclease, and the guide RNA or the nucleic acid suitable for in situ generation of such guide RNA, to produce one or more cleavages, nicks or perform editing in the target gene; wherein the AcC2C9 nuclease is directed to the target gene by its corresponding processed or unprocessed guide RNA.

In some embodiments, the gene editing method described in the present disclosure includes the following steps:
i) introducing the AcC2C9 nuclease or the polynucleotide encoding the same and the guide RNA or the polynucleotide encoding the same into a cell;
ii) under the mediation of the AcC2C9 nuclease, producing one or more nicks in the target gene, or targeting, editing, modifying or manipulating the target gene.

Each parameter in the gene editing method described in the present disclosure can be adjusted according to conventional knowledge in the art. For example, the concentration of the expression vector including the nuclease and guide RNA is preferably 1 µg; the editing is preferably 72 hours later the cell transfection.

In the gene editing method described in the present disclosure, the AcC2C9 nuclease is guided to a target gene by a processed or unprocessed guide RNA. The AcC2C9 nuclease and the guide RNA form a complex to recognize a PAM sequence upstream of the target gene. In some preferred embodiments, the method further includes introducing a donor template comprising a heterologous polynucleotide sequence into the cell.

The present disclosure also provides a use of the guide RNA, the isolated polynucleotide, the construct, the expression system, the gene editing system, the pharmaceutical composition or the method as described above in gene editing of a target gene and/or a related polypeptide thereof in an *in vivo, ex vivo* cell or cell-free environment. The ex vivo cell is at least one of bacterial cells, archaea cells, fungal cells, protozoan cells, virus cells, plant cells and animal cells. The gene editing is selected from the group consisting of gene cleavage, gene deletion, gene insertion, point mutation, transcription repression, transcription activation, base editing, and guided editing, including but not limited to:
cleaving a target gene;
manipulating the expression of a target gene;
genetically modifying a target gene;
genetically modifying a target gene-related polypeptide;
intentional and controlled damage at any desired position of a target gene;
intentional and controlled repair at any desired position of the target gene; and
modifying a target gene by means other than introducing double-stranded breakage (the AcC2C9 nuclease has an enzymatic activity, which allows it to modify a target gene by means other than introducing double-stranded breakage; the enzymatic activity can be owned by AcC2C9 itself, or obtained by, for example, fusing a heterologous polypeptide with an enzymatic activity to the AcC2C9 nuclease to form a chimeric AcC2C9 nuclease, the enzymatic activity includes but is not limited to methyltransferase activity, deamination activity, dismutase activity, alkylation activity, demethylase activity, DNA repair activity, transposase activity, recombinase activity, DNA damage activity, depurinization activity, oxidation activity, pyrimidine dimer formation activity, etc.).

Preferably, the gene editing is gene deletion or gene cleavage; the gene editing can be used to achieve one or more of the following, including but not limited to the correction of pathogenic sites, gene function research, cell function enhancement, cell therapy, etc.

The AcC2C9 nuclease or a polynucleotide encoding the same, the guide RNA or a polynucleotide encoding the same, the recombinant expression vector, the system, the composition, and the kit can be applied to research field, diagnosis field, industry field (e.g., microbial engineering), drug development (e.g., high-throughput screening), target confirmation, imaging field, treatment field, etc.

In some embodiments, the target gene is a target DNA. In some embodiments, the target DNA can be a naked DNA *in vitro* that is not bound to DNA-related proteins. In some embodiments, the target DNA is a chromosomal DNA in cells *in vitro.* In some embodiments, the target gene is a target RNA. In some embodiments, enabling the target DNA to contact with a targeting complex comprising the AcC2C9 nuclease and the guide RNA, and the guide RNA that comprises a nucleotide sequence complementary to the target DNA provides target specificity for the targeting complex; the AcC2C9 nuclease provides site-specific activity. In some embodiments, the targeting complex modifies a target DNA, leading to, for example, DNA cleavage, DNA methylation, DNA damage, DNA repair, etc. In some embodiments, the targeting complex modifies a target DNA-related polypeptide (e.g., histone, a DNA-binding protein, etc.), thereby leading to methylation, histone acetylation, histone ubiquitination, etc. of the target DNA-related polypeptide-histone, for example.

In the method described in the present disclosure, an AcC2C9 nuclease or a nucleic acid comprising a nucleotide sequence encoding a polypeptide of the AcC2C9 nuclease can be introduced into a cell by a commonly known method. Similarly, a guide RNA or a nucleic acid comprising a nucleotide sequence encoding the guide RNA can be introduced into a cell by a commonly known method. The commonly known methods include DEAE-dextran mediated transfection, liposome mediated transfection, virus or phage infection, lipid transfection, transfection, conjugation, protoplast fusion, polyethyleneimine mediated transfection, electroporation, calcium phosphate precipitation, gene gun, calcium chloride precipitation, microinjection, nanoparticle mediated nucleic acid delivery, etc. For example, electroporation, calcium chloride precipitation, microinjection, lipid transfection and the like are used for plasmid delivery. For viral vector delivery, enabling cells to contact with viral particles comprising donor polynucleotides and/or nucleic acids encoding a guide RNA and/or an AcC2C9 nuclease and/or a chimeric AcC2C9 nuclease.

In some embodiments, in the method described in the present disclosure, a nuclease cleaves a target DNA in a cell to generate double-stranded breakage, and then the cell usually performs repair in the following ways: non-homologous end joining (NHEJ) and homology-directed repair.

**The** present disclosure also provides a cell, wherein the cell includes those have been genetically modified with the AcC2C9 nuclease or a polynucleotide encoding the same, the guide RNA or a polynucleotide encoding the same, the recombinant expression vector, the system and the composition as described above, or those undergo gene editing with the above gene editing system or the method.

In the present disclosure, the effective dosage of the guide RNA and/or the AcC2C9 nuclease and/or the recombinant expression vector and/or the donor polynucleotide is known for those skilled in the art. It can be determined according to different routes of administration and the characteristics of the disorders to be treated.

In the present disclosure, the bacteria or prokaryotic bacteria can be *E. coli, Klebsiella pneumoniae, Bacteroides ovatus, Campylobacter jejuni, Staphylococcus saprophyticus, Enterococcus faecalis, Bacteroides thetaiotaomicron, Bacteroides vulgatus, Bacteroides uniformis, Lactobacillus casei, Bacteroides fragilis, Acinetobacter Iwoffii, Fusobacterium nucleatum, Bacteroides johnsonii, Bacteroides arabidopsis, Lactobacillus rhamnosus, Bacteroides massiliensis, Parabacteroides merdae, Fusobacterium mortiferum,* and *Bifidobacterium breve,* etc.

In the present disclosure, the eukaryotic cells include, but are not limited to, eukaryotic cells such as mammalian cells and fungi. The fungi include yeasts and *Aspergillus,* for example, *Saccharomyces cerevisiae, Hansenula polymorpha, Pichia pastoris, Kluyveromyces fragilis, Kluyveromyces lactis, Schizosaccharomyces pombe, Candida albicans, Candida dubliniensis, Candida glabrata, Candida guilliermondii, Candida kefyr, Candida krusei, Candida lusitaniae, Candida melinii, Candida oleophila, Candida parapsilosis, Candida tropicalis, Candida utilis, Aspergillus fumigatus, Aspergillus flavus, Aspergillus niger, Aspergillus clavatus, Aspergillus glaucus, Aspergillus nidulans, Aspergillus oryzae, Aspergillus terreus, Aspergillus ustus,* and *Aspergillus versicolor,* etc.

In the examples of the present disclosure, a novel genome editing method based on a small-sized CRISPR/AcC2C9 nuclease is disclosed. The present disclosure shows that AcC2C9 can accurately cleave genomic DNA and achieve double-stranded breakage of the genomic DNA through the guiding function of a guide RNA. The system can efficiently and accurately achieve gene editing in a living cell by using the host cell's own or exogenous repair mechanism.

The present disclosure also provides a method for preparing the guide RNA as described above, including modification of tracrRNA or crRNA of the RNA framework alone and modification of tracrRNA and or crRNA of the RNA framework in combination, and the modification is selected from truncation, addition, or substitution of one or more nucleotides in tracrRNA or crRNA, or linking tracrRNA and crRNA through a linker to prepare the guide RNA.

Parts of the sequences described in the present disclosure are as follows:
The AcC2C9 nuclease described in the present disclosure is *Actinomadura craniellae* C2C9 (AcC2C9), and its amino acid sequence preferably comprises the sequence shown below: 
MVQTEILKAFRFALDPTSVQVAALSRHAGAARWAFNHALAAKVGAHERWRA
EVAKLVGDGVPEEQARRQVRVPVPMKPAIQKALNAVKGDSRKGLDGACPWWHEV
NTYAFQSAFIDADQAWKNWLDSLSGKRVGRRVGYPRFKKKGRARDSFRLHHDVKK
PSIRLAGYRRLRLPRIGEVRLHDSGKRLARLIDRGDAVVQSVTVSRGGHRWYASVL
CKVTVQVPDRPSRRQRERGAVGVDLGVKVLAALSKPLVVDDPSSALVRNPQHLRQ
AERRLLKAQRALARTQKGSARREKAKRRVGRAHHEVAVRRHAALHQITKRLTTGFA
 
VVALEDLNVAGMTRSARGTVAAPGKNVRQKAGLNRVILDSAPAELRRQVNYKATW
YGSELAVADRWFPSSKTCSGCGWQNPHLKLSDRVFRCTDCGLVMDRAMNAARNI
ERHAVLIDRNVACDRRETLNARGASIRPTTSRGGRHDASKREGSGARPESPQQSD
LLTLPTLNNETATPP*(SEQ ID NO. 1);
*E*. *coli* codon-optimized nucleotide sequence encoding the AcC2C9 nuclease comprises the following sequence: 
5'ATGGTGCAGACGGAGATCCTGAAGGCGTTTCGGTTTGCGTTGGATCCG
ACGTCTGTGCAGGTTGCTGCCCTTTCCCGGCATGCTGGTGCGGCCAGGTGGGC
GTTCAACCATGCTCTCGCGGCCAAGGTCGGCGCGCATGAGCGGTGGCGGGCG
GAGGTAGCCAAGCTTGTCGGGGACGGTGTTCCTGAGGAGCAGGCTCGTAGGC
AGGTGCGCGTCCCGGTTCCGATGAAGCCGGCGATCCAGAAGGCGTTGAACGC
GGTCAAGGGTGACTCTCGCAAGGGCTTGGATGGTGCGTGCCCGTGGTGGCAT
GAGGTCAACACTTACGCGTTCCAGTCCGCTTTCATCGACGCGGACCAGGCGTG
GAAGAACTGGTTGGACTCGTTGTCGGGGAAGCGGGTCGGCCGCCGGGTGGGG
TACCCGCGGTTCAAGAAGAAGGGGCGGGCCCGCGACTCGTTTCGCTTGCACCA
CGACGTGAAGAAGCCAAGCATCCGGCTGGCCGGGTACCGGCGGTTGCGGCTG
CCGAGGATCGGGGAAGTCCGGCTTCATGACTCGGGGAAGCGTCTGGCACGGC
TGATCGACCGAGGTGACGCGGTGGTGCAGTCGGTGACGGTGTCGCGGGGTGG
CCATCGCTGGTATGCGTCTGTGCTGTGCAAGGTCACCGTGCAGGTACCGGATC
GGCCGTCACGGCGCCAGCGGGAACGAGGCGCTGTTGGGGTGGACCTTGGCGT
TAAGGTCCTCGCGGCTTTGTCCAAGCCACTGGTGGTCGACGACCCGTCGAGTG
CCCTGGTGCGGAATCCGCAGCATCTGCGGCAGGCCGAGCGGCGTCTACTGAA
GGCTCAGCGGGCGTTGGCGCGTACACAAAAAGGTTCAGCCCGACGCGAGAAG
GCCAAGCGGCGAGTTGGTCGCGCGCATCACGAGGTTGCTGTGCGCCGGCATG
CTGCGCTCCATCAGATCACCAAGCGCCTGACCACCGGCTTCGCGGTCGTCGCG
CTTGAGGATCTGAACGTGGCCGGTATGACAAGGTCAGCGCGGGGAACAGTGGC
AGCGCCGGGAAAGAACGTGCGGCAGAAGGCAGGCCTGAACCGCGTCATCCTG
GACTCCGCTCCAGCCGAGTTGCGTCGCCAGGTGAACTACAAGGCCACCTGGTA
TGGATCTGAGCTGGCGGTCGCAGACCGCTGGTTCCCCTCGAGTAAGACCTGTT
 
CGGGTTGCGGATGGCAAAACCCACACCTGAAGCTGTCCGACAGGGTGTTTCGT
TGCACAGACTGCGGGCTGGTGATGGACCGGGcCATGAACGCGGCCCGGAACAT
CGAACGCCACGCAGTGCTGATCGATCGAAACGTCGCCTGCGATAGGCGGGAGA
CGCTAAACGCCCGTGGAGCATCCATAAGACCAACCACCTCTCGGGGCGGTAGG
CACGACGCATCGAAGCGGGAAGGCTCCGGCGCTCGGCCTGAGTCACCTCAGC
AGAGTGATCTGCTGACGCTCCCAACCTTGAATAACGAAACGGCAACGCCTCCAT
AA-3' (SEQ ID NO. 2);
human codon-optimized nucleotide sequence encoding the AcC2C9 nuclease comprises the following sequence: 
5'ATGGTGCAGACAGAGATCCTGAAGGCCTTTCGGTTCGCCCTCGATCCT
ACTTCCGTCCAGGTGGCCGCCCTGAGCCGGCACGCCGGGGCCGCCAGGTGGG
CCTTCAACCACGCCCTCGCCGCCAAGGTGGGGGCCCACGAGCGGTGGCGGGC
CGAGGTGGCCAAGCTCGTGGGCGATGGCGTGCCCGAGGAGCAGGCCCGGCG
GCAGGTCCGGGTCCCCGTGCCTATGAAGCCAGCCATCCAGAAGGCCCTGAACG
CCGTGAAGGGGGATTCCCGGAAGGGCCTGGATGGGGCCTGCCCATGGTGGCA
CGAGGTGAACACCTACGCCTTCCAGAGCGCCTTTATTGACGCCGACCAGGCAT
GGAAGAACTGGCTCGACAGCCTGAGCGGCAAGAGAGTGGGGAGGAGGGTGGG
CTACCCCCGGTTTAAGAAGAAGGGCCGGGCTCGGGATAGCTTTAGATTGCACC
ACGATGTGAAGAAGCCCAGCATCAGGCTGGCTGGCTACAGAAGGCTGAGGCTG
CCTAGGATTGGCGAGGTGAGGCTGCACGACAGCGGCAAGCGGCTCGCCAGGC
TCATTGACAGAGGCGACGCCGTGGTGCAGTCCGTGACTGTGAGTAGGGGCGG
ACACAGGTGGTACGCCTCCGTGCTCTGCAAGGTGACAGTGCAGGTGCCCGATC
GGCCCAGCAGGCGGCAGCGGGAGAGGGGCGCCGTGGGAGTGGACCTGGGGG
TGAAGGTGCTGGCCGCCCTGTCCAAGCCCCTGGTCGTCGACGACCCAAGCAGC
GCCCTGGTGAGAAACCCCCAGCACCTGAGGCAGGCTGAGAGGCGGCTGCTCA
AGGCCCAGCGGGCCCTGGCCAGGACACAGAAGGGCAGCGCCAGGAGAGAGAA
GGCCAAGCGGCGGGTGGGGAGGGCCCATCACGAGGTGGCCGTCCGGCGGCA
CGCCGCCCTGCACCAGATCACCAAGCGGCTGACCACAGGGTTCGCCGTGGTG
GCCCTGGAGGATCTGAACGTCGCCGGCATGACCAGGTCCGCCAGGGGGACAG
 
TGGCCGCCCCAGGGAAGAACGTCCGGCAGAAGGCCGGGCTGAACCGGGTCAT
CCTGGACAGCGCCCCTGCCGAGCTGCGGAGGCAGGTGAACTACAAGGCCACC
TGGTACGGGAGCGAGCTGGCCGTGGCCGACAGGTGGTTCCCCTCCAGCAAGA
CCTGCAGCGGGTGCGGCTGGCAGAACCCCCACTTGAAGCTCTCCGATAGGGTG
TTTAGGTGCACCGACTGCGGCCTGGTCATGGATAGAGATATGAACGCCGCTAG
GAACATCGAGAGGCACGCTGTGTTGATTGATAGGAACGTGGCCTGCGACCGGC
GGGAGACTTTGAACGCCAGGGGGGCCAGCATCCGGCCCACCACAAGCCGGGG
GGGGCGGCACGACGCCAGCAAGAGGGAGGGGAGCGGGGCCCGGCCAGAGTC
CCCCCAGCAGTCTGATCTGCTCACACTCCCCACACTGAACAACGAAACCGCCAC
ACCCCCC- 3' (SEQ ID NO. 3);
different versions of guide RNA sequences for the AcC2C9 nuclease described in the present disclosure are shown as follows, in which the light gray shows the RNA framework sequence, and if there is a linker in the guide RNA, the linker sequence will be GAAA, with the linker as part of the framework; the dotted underline shows tracrRNA; the double underline shows the sequence of the tracr duplex; the underlined shows sequence of the gene targeting section; the wavy line shows the RNA structure stabilizing sequence.

Original version of guide RNA, i.e., gRNA_M1, of CRISPR/AcC2C9 system: 
5'- GAACGCGGCCCGGAACAUCGAACGCCACGCAGUGCUGAUCGAUCGAAACGUC
GCCUGCGAUAGGCGGGAGACGCUAAACGCCCGUGGAGCAUCCAUAAGACCAA
CCACCUCUCGGGGCGGUAGGCACGACGCAUCGAAGCGGGAAGGCUCCGGCG
CUCGGCCUGAGUCACCUCAGCAGAGUGAUCUGCUGACGCUCCCAACCUUGAA
UAACGAAACGGCAACGCCUCCAUAGCGGUGCAGGUCAAUAAGGGUCGGCCCC
ACGCGUGUAGGGAGCGAUCGagugacaguauccucuguau-3' (SEQ ID NO. 4),
a schematic diagram of its secondary structure is shown in Fig. 1; wherein the nucleotide sequence of the RNA framework is set forth in SEQ ID NO. 106, the nucleotide sequence of the tracrRNA is set forth in SEQ ID NO. 117, and the nucleotide sequence of the tracr duplex is set forth in SEQ ID NO. 127.

Optimized version guide RNA sequence gRNA_M2 of CRISPR/AcC2C9 provided in the present disclosure: 
5'-
CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCAACCUUGAAUAACGAAACGGCAACGCCUCCAUAGCGGU
GCAGGUCAAUAAGGGUCGGCCCCACGCGUGUAGGGAGCGAUCGagugacaguau
ccucuguau-3' (SEQ ID NO. 5),
 wherein the nucleotide sequence of the RNA framework
is set forth in SEQ ID NO. 107, the nucleotide sequence of the tracrRNA is set forth in SEQ ID NO. 140, and the nucleotide sequence of the tracr duplex is set forth in SEQ ID NO. 127.
gRNA_M3: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCAACCUUGAAUAACGAAACGAAAGUCGGCCCCACGCGUG
UAGGGAGCGAUCGagugacaguauccucuguau-3' (SEQ ID NO. 6),
 wherein the
   nucleotide sequence of the RNA framework is set forth in SEQ ID NO. 108, the nucleotide sequence of the tracrRNA is set forth in SEQ ID NO. 118, and the nucleotide sequence of the tracr duplex is set forth in SEQ ID NO. 128.
|gRNA_M4: 5'- CAGUGCUGAUCGAUCGAAACGUCGCCUGCGAUAGGCGGGAGACGCUAAACGC CCGUGGAGCAUCCAUAAGACCAACCACCUCUCGGGGCGGUAGGCACGACGCA UCGAAGCGGGAAGGCUCCGGCGCUCGGCCUGAGUCACCUCAGCAGAGUGAU CUGCUGACGCUCCCAACCUUGAAUAACGGAAACCCCACGCGUGUAGGGAGCG AUCGagugacaguauccucuguau-3' (SEQ ID NO. 7), wherein the nucleotide sequence
   of the RNA framework is set forth in SEQ ID NO. 109, the nucleotide sequence of the tracrRNA is set forth in SEQ ID NO. 119, and the nucleotide sequence of the tracr duplex is set forth in SEQ ID NO. 129.
gRNA_M5: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCAACCUUGAAUGAAAACGCGUGUAGGGAGCGAUCGaguga
caguauccucuguau-3' (SEQ ID NO. 8),
 wherein the nucleotide sequence of the RNA
   framework is set forth in SEQ ID NO. 110, the nucleotide sequence of the tracrRNA is set forth in SEQ ID NO. 120, and the nucleotide sequence of the tracr duplex is set forth in SEQ ID NO. 130.
gRNA_M6: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCAACCUUGGAAACGUGUAGGGAGCGAUCGagugacaguaucc
ucuguau-3' (SEQ ID NO. 9),
 wherein the nucleotide sequence of the RNA framework
   is set forth in SEQ ID NO. 111, the nucleotide sequence of the tracrRNA is set forth in SEQ ID NO. 121, and the nucleotide sequence of the tracr duplex is set forth in SEQ ID NO. 131.
gRNA_M7: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCAACGAAAGUAGGGAGCGAUCGagugacaguauccucuguau-3'
(SEQ ID NO. 10),
 wherein the nucleotide sequence of the RNA framework is set forth in SEQ ID NO. 112, the nucleotide sequence of the tracrRNA is set forth in SEQ ID NO. 122, and the nucleotide sequence of the tracr duplex is set forth in SEQ ID NO. 132.
gRNA_M8: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCCCGAAAGGGAGCGAUCGagugacaguauccucuguau-3' (SEQ ID
NO. 11),
 wherein the nucleotide sequence of the RNA framework is set forth in SEQ
   ID NO. 113, the nucleotide sequence of the tracrRNA is set forth in SEQ ID NO. 123, and the nucleotide sequence of the tracr duplex is set forth in SEQ ID NO. 133.
gRNA_M9: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCUCGAAAGAGCGAUCGagugacaguauccucuguau-3' (SEQ ID NO.
12), '
 wherein the nucleotide sequence of the RNA framework is set forth in SEQ ID NO. 114, the nucleotide sequence of the tracrRNA is set forth in SEQ ID NO. 124, and the nucleotide sequence of the tracr duplex is set forth in SEQ ID NO. 134.
gRNA_M10: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGCGAAAGCGAUCGagugacaguauccucuguau-3' (SEQ ID NO. 13),
 wherein the nucleotide sequence of the RNA framework is set forth in SEQ ID NO. 115, the nucleotide sequence of the tracrRNA is set forth in SEQ ID NO. 125, and the nucleotide sequence of the tracr duplex is set forth in SEQ ID NO. 135.
gRNA_M11: 
5'- CAGUGCUGAUCGAUCGAAACGUCGCCUGCGAUAGGCGGGAGACGCUAAACGC
CCGUGGAGCAUCCAUAAGACCAACCACCUCUCGGGGCGGUAGGCACGACGCA
UCGAAGCGGGAAGGCUCCGGCGCUCGGCCUGAGUCACCUCAGCAGAGUGAU
CUGCUGACGAAAGAUCGagugacaguauccucuguau-3' (SEQ ID NO. 14),
 wherein the
   nucleotide sequence of the RNA framework is set forth in SEQ ID NO. 116, the nucleotide sequence of the tracrRNA is set forth in SEQ ID NO. 126, and the nucleotide sequence of the tracr duplex is set forth in SEQ ID NO. 136.
gRNA_M12: 
5'- GAACGCGGCCCGGAACAUCGAACGCCACGCAGUGCUGAUCGAUCGAAACGUC
GCCUGCGAUAGGCGGGAGACGCUAAACGCCCGUGGAGCAUCCAUAAGACCAA
CCACCUCUCGGGGCGGUAGGCACGACGCAUCGAAGCGGGAAGGCUCCGGCG
CUCGGCCUGAGUCACCUCAGCAGAGUGAUCUGCUGACGCUCCCAACCUUGAA
UAACGAAACGGCAACGCCUCCAUAGCGGUGCAGGUCAAUAAGGGUCGGCCCC
ACGCGUGUAGGGAGCGAUCGagugacaguauccucuguauUUUUAUUUUUU-3' (SEQ
ID NO. 15),
 wherein the nucleotide sequence of the RNA framework is set forth in SEQ
   ID NO. 106, the nucleotide sequence of the tracrRNA is set forth in SEQ ID NO. 117, the nucleotide sequence of the tracr duplex is set forth in SEQ ID NO. 127, and the nucleotide sequence of the RNA structure stabilizing sequence is set forth in SEQ ID NO. 137.
gRNA_M13: 
5'- GAACGCGGCCCGGAACAUCGAACGCCACGCAGUGCUGAUCGAUCGAAACGUC
GCCUGCGAUAGGCGGGAGACGCUAAACGCCCGUGGAGCAUCCAUAAGACCAA
CCACCUCUCGGGGCGGUAGGCACGACGCAUCGAAGCGGGAAGGCUCCGGCG
CUCGGCCUGAGUCACCUCAGCAGAGUGAUCUGCUGACGCUCCCAACCUUGAA
UAACGAAACGGCAACGCCUCCAUAGCGGUGCAGGUCAAUAAGGGUCGGCCCC
ACGCGUGUAGGGAGCGAUCGagugacaguauccucuguauUUGACGCGGUUCUAUC
UAGUUACGCGUUAAACCAACUAGAAA-3' (SEQ ID NO. 16),
 wherein the nucleotide
   sequence of the RNA framework is set forth in SEQ ID NO. 106, the nucleotide sequence of the tracrRNA is set forth in SEQ ID NO. 117, the nucleotide sequence of the tracr duplex is set forth in SEQ ID NO. 127, and the nucleotide sequence of the RNA structure stabilizing sequence is set forth in SEQ ID NO. 138.
gRNA_M14: 
5'- GAACGCGGCCCGGAACAUCGAACGCCACGCAGUGCUGAUCGAUCGAAACGUC
GCCUGCGAUAGGCGGGAGACGCUAAACGCCCGUGGAGCAUCCAUAAGACCAA
CCACCUCUCGGGGCGGUAGGCACGACGCAUCGAAGCGGGAAGGCUCCGGCG
CUCGGCCUGAGUCACCUCAGCAGAGUGAUCUGCUGACGCUCCCAACCUUGAA
UAACGAAACGGCAACGCCUCCAUAGCGGUGCAGGUCAAUAAGGGUCGGCCCC
ACGCGUGUAGGGAGCGAUCGagugacaguauccucuguauACAUGCGAUUGACGCG
GUUCUAUCUAGUUACGCGUUAAACCAACUAGAAA-3' (SEQ ID NO. 17),
 wherein
   the nucleotide sequence of the RNA framework is set forth in SEQ ID NO. 106, the nucleotide sequence of the tracrRNA is set forth in SEQ ID NO. 117, the nucleotide sequence of the tracr duplex is set forth in SEQ ID NO. 127, and the nucleotide sequence of the RNA structure stabilizing sequence is set forth in SEQ ID NO. 139.

The terms "AcC2C9", "AcC2C9 nuclease", "AcC2C9 polypeptide", and "AcC2C9 protein" can be used interchangeably.

The terms "guide RNA", "gRNA", "single gRNA" and "chimeric gRNA" can be used interchangeably.

The term "a" or "an" entity refers to one or more of such entities; therefore, the terms "a" (or "an"), "one or more" and "at least one" are used interchangeably herein.

The term "homology", "identity" or "similarity" refers to the sequence similarity between two peptides or two nucleic acid molecules. Homology can be determined by aligning the corresponding positions in different polypeptide or nucleic acid molecules. When the same position in the compared molecular sequence and another different sequence is occupied by the same base or amino acid, then the compared molecular sequence and another different sequence are homologous at that position. The degree of homology between sequences is determined by the function of the number of matching or positions shared by the sequences. An "unrelated" or a "non-homologous" sequence should have less than 20% homology with one of the sequences disclosed in the present disclosure.

A polynucleotide or polynucleotide region (or polypeptide or polypeptide region) has a certain percentage (e.g., 20%, 30%, 40%, 50%, 60%, 70%, 80%, 90%, 95%, 98%, or 99%) of sequence homology with another polynucleotide or polynucleotide region (or polypeptide or polypeptide region) refers to that the two sequences being aligned possess that percentage of bases (or amino acids) which are identical. The alignment and percentage homology or sequence identity can be determined using software procedures and methods known in the art.

In the present disclosure, the terms "polynucleotide" and "oligonucleotide" are used interchangeably, and they refer to polymerized forms of nucleotides (no matter deoxynucleotides or ribonucleotides or their analogues) of any length. A polynucleotide can have any three-dimensional structure and can perform any function known or unknown. Examples of polynucleotides include, but are not limited to, genes or gene fragments (including probes, primers, EST or SAGE tags), exons, introns, messenger RNA (mRNA), transport RNA, ribosomal RNA, ribozymes, cDNA, dsRNA, siRNA, miRNA, recombinant polynucleotides, branched polynucleotides, plasmids, vectors, isolated DNA of any sequence, and isolated RNA of any sequence, nucleic acid probes, and primers. Polynucleotides also comprise modified nucleotides, e.g., methylated nucleotides and nucleotide analogues. If there is a modification of a polynucleotide, the modification can be introduced either before or after the assembly of the polynucleotide. A nucleotide sequence can be interrupted by non-nucleotide components. A polynucleotide can be further modified after polymerization, for example, by coupling to a labeled component. The term refers to both double-stranded and single-stranded polynucleotide molecules. Unless otherwise specified or required, any implementation example of a polynucleotide disclosed in the present disclosure includes its double-stranded form and any one of two complementary single-stranded forms known or predicted to constitute the double-stranded structure.

When being applied to a polynucleotide, the term "encode" refers to that the polynucleotide "encodes" a polypeptide, which means that in its natural state or when being manipulated by methods commonly known to those skilled in the art, the polynucleotide can be transcribed and/or translated to produce the polypeptide of interest and/or fragments thereof, or to produce mRNA that is capable of encoding the polypeptide of interest and/or fragments thereof. An antisense strand refers to a sequence that is complementary to the polynucleotide, and from which the encoding sequence can be determined.

The term "genomic DNA" denotes the DNA of an organism genome, including the DNA of bacterial, archaeal, fungal, protozoan, viral, plant, or animal genomes.

The term "manipulating" DNA includes binding, making nicks in one strand, or cleaving two strands of DNA, or includes modifying or editing DNA or polypeptides that bind to DNA. Manipulating DNA can silence, activate or regulate the expression of RNA or the polypeptide encoded by the DNA (prevent transcription, reduce transcription activity, prevent translation, or lower translation level), or prevent or enhance the binding of polypeptides and DNA. Cleavage can be carried out by various methods, e.g., enzymatic or chemical hydrolysis of phosphodiester bonds; it can involve single-strand or double-strand cleavage; DNA cleavage can result in the generation of blunt ends or staggered ends.

The term "hybridizable", "complementary" or "substantially complementary" refers to that a nucleic acid (e.g., RNA) comprises a nucleotide sequence that enables it to noncovalently bind to another nucleic acid in a sequence-specific and antiparallel manner under appropriate *in vitro* and/or *in vivo* temperature and solution ionic strength conditions, i.e., to form Watson-Crick base pairs and/or G/U base pairs, "anneal," or "hybridize".

It can be understood in the art that the sequence of a polynucleotide does not need to be 100% complementary to the sequence of a target nucleic acid to which the polynucleotide can specifically hybridize. A polynucleotide can hybridize on one or more sections. A polynucleotide can comprise at least 70%, at least 80%, at least 90%, at least 95%, at least 99% or 100% sequence complementarity to a target region within the target nucleic acid sequence.

The terms "peptide", "polypeptide" and "protein" are used interchangeably in the present disclosure, and represent polymerized forms of amino acids of any length, which can include encoded and non-encoded amino acids, chemically or biochemically modified or derived amino acids, and polypeptides with modified peptide backbones.

The term the DNA sequence "encoding" a specific RNA is a DNA nucleic acid sequence transcribed into the RNA. A DNA polynucleotide can encode an RNA that will be translated into a protein (mRNA), or a DNA polynucleotide can encode an RNA that will not be translated into a protein (e.g., tRNA, rRNA or gRNA; also known as a "non-coding" RNA or "ncRNA"). A "protein encoding sequence" or a sequence encoding a specific protein or polypeptide is a nucleic acid sequence that is transcribed into mRNA (in the case of DNA) and translated into a polypeptide (in the case of mRNA) *in vivo* or *in vitro* under the control of an appropriate regulatory sequence.

The term "vector" or "expression vector" is a replicon, such as a plasmid, phage, virus or cosmid, to which another DNA section, i.e., an "insertion fragment", can be attached in order to achieve replication of the section in a cell.

The term "expression cassette" comprises a DNA encoding sequence operably linked to a promoter. "Operably linked" denotes parallel linking, with each component in a relationship that allows them to function in their intended way. The terms "recombinant expression vector" or "DNA construct" are used interchangeably in the present disclosure to denote a DNA molecule comprising a vector and at least one insertion fragment. A recombinant expression vector is usually produced for the purpose of expressing and/or amplifying inserted fragments or for constructing other recombinant nucleotide sequences.

When an exogenous DNA, e.g., a recombinant expression vector, has been introduced into a cell, the cell has been "genetically modified" or "transformed" or "transfected" by this DNA. The existence of the exogenous DNA leads to permanent or temporary genetic changes. The transformed DNA is integrated or not integrated into the genome of the cell.

The term "target DNA" is a DNA polynucleotide comprising "target sites" or "target sequences". The terms "target site", "target sequence", "target protospacer DNA" or "protospacer-like sequence" are used interchangeably in the present disclosure to denote the nucleic acid sequence existing in the target DNA, to which the DNA-targeting section of gRNA will bind if there is suitable condition. RNA molecules comprise sequences that bind, hybridize or are complementary to a target sequence in a target DNA, thus directing the linked polypeptide of RNA to a specific position (target sequence) in the target DNA. "Cleavage" refers to the breakage of the covalent backbone of DNA molecules.

The terms "nuclease" and "endonuclease" can be used interchangeably to refer to an enzyme with endonucleolytic catalytic activity for polynucleotide cleavage. The "cleavage domain" or "active domain" or "nuclease domain" of a nuclease refers to a polypeptide sequence or domain with catalytic activity for DNA cleavage within the nuclease. The cleavage domain can be a single polypeptide chain, or the cleavage activity can be generated by the association of two or more polypeptides.

The term "localized polypeptide" or "RNA-binding site-directed polypeptide" refers to a polypeptide that binds to RNA and targets a specific DNA sequence.

The term "guide sequence" or DNA-targeting section (or "DNA-targeting sequence") comprises a nucleotide sequence that is complementary to a specific sequence within a target DNA (i.e., the complementary strand of the target DNA), with the target DNA referred to as a "protospacer-like" sequence in the present disclosure.

The term "recombination" refers to the process of exchanging genetic information between two polynucleotides. As used in the present disclosure, "homology-directed repair (HDR)" denotes a specialized form of DNA repair that occurs, for example, during the repair of double-stranded breakage in cells. This process requires nucleotide sequence homology, utilizing a "donor" molecule to provide a template for the repair of a "target" molecule (i.e., the molecule has undergone double-stranded breakage), and resulting in the transfer of genetic information from the donor to the target. If the donor polynucleotide is different from the target molecule, and part or all of the sequences of the donor polynucleotide are incorporated into the target DNA, the repair directed by homology may lead to changes in the sequence of the target molecule (e.g., insertion, deletion and mutation).

The term "non-homologous end joining (NHEJ)" refers to the repair of double-stranded breakage in DNA by directly joining the broken ends to each other without the need for a homologous template. NHEJ often leads to the deletion of nucleotide sequences near the site undergone double-stranded breakage.

The term "treatment" includes preventing the occurrence of a disease or symptom; inhibiting a disease or symptom; or alleviating a disease.

The terms "individual," "subject," "host," and "patient" are used interchangeably in the present disclosure and denote any mammalian subject, particularly a human, for whom diagnosis, treatment, or therapy is desired.

The embodiments of the present disclosure are illustrated below through specific examples, and those skilled in the art can easily understand other advantages and effects of the present disclosure from the contents disclosed in this specification. The present disclosure can also be implemented or applied through other different detailed embodiments, and various details in this specification can be modified or changed based on different viewpoints and applications without departing from the spirit of the present disclosure.

Before further describing the detailed embodiments of the present disclosure, it should be understood that the protection scope of the present disclosure is not limited to the following specific implementation examples; also, it should be understood that the terms used in the examples of the present disclosure are for the purpose of describing specific implementation examples, and not for the purpose of limiting the protection scope of the present disclosure; after reading the present disclosure, those skilled in the art can make various changes or modifications to the present disclosure, and these equivalent forms also fall within the scope defined by the claims in the present disclosure. For example, a single-gene editing system based on AcC2C9 is developed by fusing an engineered inactivated AcC2C9 with a base deaminase; a Prime editing system based on AcC2C9 is developed by fusing an inactivated AcC2C9 with a reverse transcriptase; a transcription activation system based on AcC2C9 is developed by fusing an inactivated AcC2C9 with a transcription activation factor; an epigenetic modification system based on AcC2C9 is developed by fusing an inactivated AcC2C9 with a nucleic acid epigenetic modifying enzyme; and a transcription repression system based on AcC2C9 is developed by utilizing an inactivated AcC2C9. In the specification and claims of the present disclosure, the singular forms "a", "an" and "the" include the plural forms unless the context clearly indicates otherwise.

When the examples give numerical ranges, it should be understood that unless otherwise specified in the present disclosure, two endpoints of each numerical range and any numerical value between the two endpoints can be selected. Unless otherwise defined, all technical and scientific terms used in the present disclosure have the same meaning as commonly understood by those skilled in the art. In addition to the specific methods, equipment and materials used in the examples, according to the knowledge of the prior art by those skilled in the art and the records of the present disclosure, any methods, equipment and materials in the prior art similar to or equivalent to the methods, equipment and materials described in the examples of the present disclosure can be used to achieve the present disclosure.

In the examples of the present disclosure, the mainly used CRISPR system is the V-U4 type CRISPR system, wherein the effector protein is mainly the *Actinomadura craniellae* C2C9 (AcC2C9) nuclease. The AcC2C9 nuclease can be accurately directed to the target gene under the guidance of the corresponding guide RNA, cleave the genomic DNA, and achieve the double-stranded breakage of the genomic DNA. The system can efficiently and accurately achieve gene editing in a living cell by using the host cell's own or exogenous repair mechanism.

Where the specific technology or conditions are not specified in the examples, it should be understood that they are carried out according to the conventional technology or conditions described in the literature in the art or according to the product manufacturer's instructions.

The primers used in the examples were synthesized by Shanghai Sangon Biotech Co., Ltd. and Suzhou Genewiz Biotechnology Co., Ltd. Any reagents or instruments used without specifying the manufacturer are considered products that are commercially available.

### Example 1 Engineering of corresponding guide RNAs for AcC2C9 Nuclease

In this example, the corresponding guide RNAs for the AcC2C9 nuclease were modified based on an original version gRNA_M1. The specific modification sites and the corresponding base lengths are illustrated in Fig. 1. The specific engineering methods and the lengths of the modified bases are shown in the following:
encoding nucleotide of gRNA_M1: 5'-GAACGCGGCCCGGAACATCGAACGCCACGCAGTGCTGATCGATCGAAACGTCG CCTGCGATAGGCGGGAGACGCTAAACGCCCGTGGAGCATCCATAAGACCAACC ACCTCTCGGGGCGGTAGGCACGACGCATCGAAGCGGGAAGGCTCCGGCGCTC GGCCTGAGTCACCTCAGCAGAGTGATCTGCTGACGCTCCCAACCTTGAATAACG AAACGGCAACGCCTCCATAGCGGTGCAGGTCAATAAGGGTCGGCCCCACGCGT GTAGGGAGCGATCGaagtgacagtatcctctgtat-3' (SEQ ID NO. 92)
encoding nucleotide of gRNA_M2, based on M1 version, but with 30 nt bases of stem-loop 1 removed: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACCTTGAATAACGAAACGAAAGTCGGCCCCACGCGTGTAGG GAGCGATCGaagtgacagtatcctctgtat-3' (SEQ ID NO. 93)
encoding nucleotide of gRNA_M3, based on M2 version, but with 34 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACCTTGAATAACGGAAACCCCACGCGTGTAGGGAGCGATCGa agtgacagtatcctctgtat-3' (SEQ ID NO. 94)
encoding nucleotide of gRNA_M4, based on M2 version, but with 43 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACCTTGAATAACGGAAACCCCACGCGTGTAGGGAGCGATCGa agtgacagtatcctctgtat-3' (SEQ ID NO. 95)
encoding nucleotide of gRNA_M5, based on M2 version, but with 51 nt bases of upper part of stem-loop 6removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACCTTGAATGAAAACGCGTGTAGGGAGCGATCGaagtgacagtatc ctctgtat-3' (SEQ ID NO. 96)
encoding nucleotide of gRNA_M6, based on M2 version, but with 57 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACCTTGGAAACGTGTAGGGAGCGATCGaagtgacagtatcctctgtat-3' (SEQ ID NO. 97)
encoding nucleotide of gRNA_M7, based on M2 version, but with 64 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACGAAAGTAGGGAGCGATCGaagtgacagtatcctctgtat- 3' (SEQ ID NO. 98)
encoding nucleotide of gRNA_M8, based on M2 version, but with 70 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCGAAAGGGAGCGATCGaagtgacagtatcctctgtat-3' (SEQ ID NO. 99)
encoding nucleotide of gRNA_M9, based on M2 version, but with 74 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCGAAAGAGCGATCGaagtgacagtatcctctgtat-3' (SEQ ID NO. 100)
encoding nucleotide of gRNA_M10, based on M2 version, but with 78 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCGAAAGCGATCGaagtgacagtatcctctgtat-3' (SEQ ID NO. 101)
encoding nucleotide of gRNA_M11, based on M2 version, but with 82 nt bases of upper part of stem-loop 6 removed, and four nucleotides GAAA added at truncated position for linking: 5'-CAGTGCTGATCGATCGAAACGTCGCCTGCGATAGGCGGGAGACGCTAAACGCC CGTGGAGCATCCATAAGACCAACCACCTCTCGGGGCGGTAGGCACGACGCATC GAAGCGGGAAGGCTCCGGCGCTCGGCCTGAGTCACCTCAGCAGAGTGATCTG CTGACGCTCCCAACGAAAGTAGGGAGCGATCGaagtgacagtatcctctgtat- 3' (SEQ ID NO. 102)
encoding nucleotide of gRNA_M12, based on M1 version, but with TTTTATTTTTT added at 3' end: 5'-GAACGCGGCCCGGAACATCGAACGCCACGCAGTGCTGATCGATCGAAACGTCG CCTGCGATAGGCGGGAGACGCTAAACGCCCGTGGAGCATCCATAAGACCAACC ACCTCTCGGGGCGGTAGGCACGACGCATCGAAGCGGGAAGGCTCCGGCGCTC GGCCTGAGTCACCTCAGCAGAGTGATCTGCTGACGCTCCCAACCTTGAATAACG AAACGGCAACGCCTCCATAGCGGTGCAGGTCAATAAGGGTCGGCCCCACGCGT GTAGGGAGCGATCGaagtgacagtatcctctgtatTTTTATTTTTT-3' (SEQ ID NO. 103)
encoding nucleotide of gRNA_M13, based on M1 version, but with TTGACGCGGTTCTATCTAGTTACGCGTTAAACCAACTAGAAA added at 3' end: 5'-GAACGCGGCCCGGAACATCGAACGCCACGCAGTGCTGATCGATCGAAACGTCG CCTGCGATAGGCGGGAGACGCTAAACGCCCGTGGAGCATCCATAAGACCAACC ACCTCTCGGGGCGGTAGGCACGACGCATCGAAGCGGGAAGGCTCCGGCGCTC GGCCTGAGTCACCTCAGCAGAGTGATCTGCTGACGCTCCCAACCTTGAATAACG AAACGGCAACGCCTCCATAGCGGTGCAGGTCAATAAGGGTCGGCCCCACGCGT GTAGGGAGCGATCGaagtgacagtatcctctgtatTTGACGCGGTTCTATCTAGTTACGCGT TAAACCAACTAGAAA-3' (SEQ ID NO. 104)
encoding nucleotide of gRNA_M14, based on M1 version, but with ACATGCGATTGACGCGGTTCTATCTAGTTACGCGTTAAACCAACTAGAAA added at 3' end: 5'-GAACGCGGCCCGGAACATCGAACGCCACGCAGTGCTGATCGATCGAAACGTCG CCTGCGATAGGCGGGAGACGCTAAACGCCCGTGGAGCATCCATAAGACCAACC ACCTCTCGGGGCGGTAGGCACGACGCATCGAAGCGGGAAGGCTCCGGCGCTC GGCCTGAGTCACCTCAGCAGAGTGATCTGCTGACGCTCCCAACCTTGAATAACG AAACGGCAACGCCTCCATAGCGGTGCAGGTCAATAAGGGTCGGCCCCACGCGT GTAGGGAGCGATCGaagtgacagtatcctctgtatACATGCGATTGACGCGGTTCTATCTAG TTACGCGTTAAACCAACTAGAAA-3' (SEQ ID NO. 105)

Accordingly, the nucleotide sequences of the guide RNAs are set forth in SEQ ID NO. 4-SEQ ID NO. 17. The underlined part represents the gene targeting section, which is a fragment with a length of 20 bp downstream of the preferred PAM sequences. In this example, human embryonic kidney cell HEK293T is used as the experimental cell.

### 1. Construction of pAcC2C9hs-M1-G1 plasmid

### 1.1 Construction of pAcC2C9hs-M1-NSP plasmid (without gene targeting section)

The following sequences were synthesized by Sangon Biotech (Shanghai) Co., Ltd.:
expression cassette of human codon-optimized gene encoding AcC2C9: 5'-ggtgaagggggcggccgctcgaggctagtctcgtgatcgataccgtcgagatccgttcactaatcggtacctatcgat agagaaatgttctggcacctgcacttgcactggggacagcctattttgctagtttgttttgtttcgttttgttttgatggagagc gtatgttagtactatcgattcttgaccgacaattgcatgaagaatctgcttagggttaggcgttttgcgctgcttcgcgatgt acgggccagatatacgcgttgacattgattattgactagttattaatagtaatcaattacggggtcattagttcatagccca tatatggagttccgcgttacataacttacggtaaatggcccgcctggctgaccgcccaacgacccccgcccattgacgt caataatgacgtatgttcccatagtaacgccaatagggactttccattgacgtcaatgggtggagtatttacggtaaact gcccacttggcagtacatcaagtgtatcatatgccaagtacgccccctattgacgtcaatgacggtaaatggcccgcct ggcattatgcccagtacatgaccttatgggactttcctacttggcagtacatctacgtattagtcatcgctattaccatggtg atgcggttttggcagtacatcaatgggcgtggatagcggtttgactcacggggatttccaagtctccaccccattgacgt caatgggagtttgttttggcaccaaaatcaacgggactttccaaaatgtcgtaacaactccgccccattgacgcaaatg ggcggtaggcgtgtacggtgggaggtctatataagcagagctggtttagtgaaccgtcagatccgctagagatccgc ggccgctaatacgactcactatagggagagccgccaccatgGCCCCTAAGAAGAAGAGGAAGGTC ATGGTGCAGACAGAGATCCTGAAGGCCTTTCGGTTCGCCCTCGATCCTACTTCC GTCCAGGTGGCCGCCCTGAGCCGGCACGCCGGGGCCGCCAGGTGGGCCTTCA ACCACGCCCTCGCCGCCAAGGTGGGGGCCCACGAGCGGTGGCGGGCCGAGG TGGCCAAGCTCGTGGGCGATGGCGTGCCCGAGGAGCAGGCCCGGCGGCAGG TCCGGGTCCCCGTGCCTATGAAGCCAGCCATCCAGAAGGCCCTGAACGCCGTG AAGGGGGATTCCCGGAAGGGCCTGGATGGGGCCTGCCCATGGTGGCACGAGG TGAACACCTACGCCTTCCAGAGCGCCTTTATTGACGCCGACCAGGCATGGAAG AACTGGCTCGACAGCCTGAGCGGCAAGAGAGTGGGGAGGAGGGTGGGCTACC CCCGGTTTAAGAAGAAGGGCCGGGCTCGGGATAGCTTTAGATTGCACCACGAT GTGAAGAAGCCCAGCATCAGGCTGGCTGGCTACAGAAGGCTGAGGCTGCCTAG GATTGGCGAGGTGAGGCTGCACGACAGCGGCAAGCGGCTCGCCAGGCTCATT GACAGAGGCGACGCCGTGGTGCAGTCCGTGACTGTGAGTAGGGGCGGACACA GGTGGTACGCCTCCGTGCTCTGCAAGGTGACAGTGCAGGTGCCCGATCGGCC CAGCAGGCGGCAGCGGGAGAGGGGCGCCGTGGGAGTGGACCTGGGGGTGAA GGTGCTGGCCGCCCTGTCCAAGCCCCTGGTCGTCGACGACCCAAGCAGCGCC CTGGTGAGAAACCCCCAGCACCTGAGGCAGGCTGAGAGGCGGCTGCTCAAGG CCCAGCGGGCCCTGGCCAGGACACAGAAGGGCAGCGCCAGGAGAGAGAAGG CCAAGCGGCGGGTGGGGAGGGCCCATCACGAGGTGGCCGTCCGGCGGCACG CCGCCCTGCACCAGATCACCAAGCGGCTGACCACAGGGTTCGCCGTGGTGGC CCTGGAGGATCTGAACGTCGCCGGCATGACCAGGTCCGCCAGGGGGACAGTG GCCGCCCCAGGGAAGAACGTCCGGCAGAAGGCCGGGCTGAACCGGGTCATCC TGGACAGCGCCCCTGCCGAGCTGCGGAGGCAGGTGAACTACAAGGCCACCTG GTACGGGAGCGAGCTGGCCGTGGCCGACAGGTGGTTCCCCTCCAGCAAGACC TGCAGCGGGTGCGGCTGGCAGAACCCCCACTTGAAGCTCTCCGATAGGGTGTT TAGGTGCACCGACTGCGGCCTGGTCATGGATAGAGATATGAACGCCGCTAGGA ACATCGAGAGGCACGCTGTGTTGATTGATAGGAACGTGGCCTGCGACCGGCGG GAGACTTTGAACGCCAGGGGGGCCAGCATCCGGCCCACCACAAGCCGGGGGG GGCGGCACGACGCCAGCAAGAGGGAGGGGAGCGGGGCCCGGCCAGAGTCCC CCCAGCAGTCTGATCTGCTCACACTCCCCACACTGAACAACGAAACCGCCACAC CCCCCtctggtggttctcccaagaagaagaggaaagtctaaccggtcatcatcaccatcaccat-3' (SEQ ID NO. 18);
expression cassette of corresponding guide RNA gRNA_M1 for AcC2C9 in human cells: 5'-gagggcctatttcccatgattccttcatatttgcatatacgatacaaggctgttagagagataattggaattaatttgactgt aaacacaaagatattagtacaaaatacgtgacgtagaaagtaataatttcttgggtagtttgcagttttaaaattatgtttta aaatggactatcatatgcttaccgtaacttgaaagtatttcgatttcttggctttatatatcttgtggaaaggacgaaacacc GGAACGCGGCCCGGAACATCGAACGCCACGCAGTGCTGATCGATCGAAACGTC GCCTGCGATAGGCGGGAGACGCTAAACGCCCGTGGAGCATCCATAAGACCAAC CACCTCTCGGGGCGGTAGGCACGACGCATCGAAGCGGGAAGGCTCCGGCGCT CGGCCTGAGTCACCTCAGCAGAGTGATCTGCTGACGCTCCCAACCTTGAATAAC GAAACGGCAACGCCTCCATAGCGGTGCAGGTCAATAAGGGTCGGCCCCACGC GTGTAGGGAGCGATCGcgagaccattggtctcatttttttgaattctcgacctcgagacaaatggcagtattca tccacaattttaaaagaaaaggggggattggggggtacagtgcaggggaaagaatagtagacataatagcaacag acatacaaactaaagaattacaaaaacaaattacaaaaattcaaaattttcgggtttattacagggacagcagagatc cactttggccgcggctcgag-3' (SEQ **ID** NO. 19);
expression cassette of puromycin resistance gene: 5'-ggggttggggttgcgccttttccaaggcagccctgggtttgcgcagggacgcggctgctctgggcgtggttccgggaa acgcagcggcgccgaccctgggactcgcacattcttcacgtccgttcgcagcgtcacccggatcttcgccgctaccctt gtgggccccccggcgacgcttcctgctccgcccctaagtcgggaaggttccttgcggttcgcggcgtgccggacgtga caaacggaagccgcacgtctcactagtaccctcgcagacggacagcgccagggagcaatggcagcgcgccgac cgcgatgggctgtggccaatagcggctgctcagcagggcgcgccgagagcagcggccgggaaggggcggtgcg ggaggcggggtgtggggcggtagtgtgggccctgttcctgcccgcgcggtgttccgcattctgcaagcctccggagcg cacgtcggcagtcggctccctcgttgaccgaatcaccgacctctctccccagggggatccaccggagcttaccatgac cgagtacaagcccacggtgcgcctcgccacccgcgacgacgtccccagggccgtacgcaccctcgccgccgcgtt cgccgactaccccgccacgcgccacaccgtcgatccggaccgccacatcgagcgggtcaccgagctgcaagaac tcttcctcacgcgcgtcgggctcgacatcggcaaggtgtgggtcgcggacgacggcgccgcggtggcggtctggac cacgccggagagcgtcgaagcgggggcggtgttcgccgagatcggcccgcgcatggccgagttgagcggttcccg gctggccgcgcagcaacagatggaaggcctcctggcgccgcaccggcccaaggagcccgcgtggttcctggcca ccgtcggcgtctcgcccgaccaccagggcaagggtctgggcagcgccgtcgtgctccccggagtggaggcggccg agcgcgccggggtgcccgccttcctggaaacctccgcgccccgcaacctccccttctacgagcggctcggcttcacc gtcaccgccgacgtcgaggtgcccgaaggaccgcgcacctggtgcatgacccgcaagcccggtgcctgacgccc gccccacgacccgcagcgcccgaccgaaaggagcgcacgaccccatgcatcggtacctttaagaccaatgactta caaggcagctgtagatcttagccactttctagagtcggggcggccggccgcttcgagcagacatga-3' (SEQ ID NO. 20);
human transient expression plasmid backbone: 5'-taagatacattgatgagtttggacaaaccacaactagaatgcagtgaaaaaaatgctttatttgtgaaatttgtgatgcta ttgctttatttgtaaccattataagctgcaataaacaagttaacaacaacaattgcattcattttatgtttcaggttcaggggg aggtgtgggaggttttttaaagcaagtaaaacctctacaaatgtggtaaaatcgataaggatccgtcgaccgatgccct tgagagccttcaacccagtcagctccttccggtgggcgcggggcatgactatcgtcgccgcacttatgactgtcttcttta tcatgcaactcgtaggacaggtgccggcagcgctcttccgcttcctcgctcactgactcgctgcgctcggtcgttcggct gcggcgagcggtatcagctcactcaaaggcggtaatacggttatccacagaatcaggggataacgcaggaaagaa catgtgagcaaaaggccagcaaaaggccaggaaccgtaaaaaggccgcgttgctggcgtttttccataggctccgc ccccctgacgagcatcacaaaaatcgacgctcaagtcagaggtggcgaaacccgacaggactataaagatacca ggcgtttccccctggaagctccctcgtgcgctctcctgttccgaccctgccgcttaccggatacctgtccgcctttctccctt cgggaagcgtggcgctttctcaatgctcacgctgtaggtatctcagttcggtgtaggtcgttcgctccaagctgggctgtg tgcacgaaccccccgttcagcccgaccgctgcgccttatccggtaactatcgtcttgagtccaacccggtaagacacg acttatcgccactggcagcagccactggtaacaggattagcagagcgaggtatgtaggcggtgctacagagttcttga agtggtggcctaactacggctacactagaaggacagtatttggtatctgcgctctgctgaagccagttaccttcggaaa aagagttggtagctcttgatccggcaaacaaaccaccgctggtagcggtggtttttttgtttgcaagcagcagattacgc gcagaaaaaaaggatctcaagaagatcctttgatcttttctacggggtctgacgctcagtggaacgaaaactcacgtta agggattttggtcatgagattatcaaaaaggatcttcacctagatccttttaaattaaaaatgaagttttaaatcaatctaa agtatatatgagtaaacttggtctgacagttaccaatgcttaatcagtgaggcacctatctcagcgatctgtctatttcgttc atccatagttgcctgactccccgtcgtgtagataactacgatacgggagggcttaccatctggccccagtgctgcaatg ataccgcgggacccacgctcaccggctccagatttatcagcaataaaccagccagccggaagggccgagcgcag aagtggtcctgcaactttatccgcctccatccagtctattaattgttgccgggaagctagagtaagtagttcgccagttaat agtttgcgcaacgttgttgccattgctacaggcatcgtggtgtcacgctcgtcgtttggtatggcttcattcagctccggttcc caacgatcaaggcgagttacatgatcccccatgttgtgcaaaaaagcggttagctccttcggtcctccgatcgttgtcag aagtaagttggccgcagtgttatcactcatggttatggcagcactgcataattctcttactgtcatgccatccgtaagatgc ttttctgtgactggtgagtactcaaccaagtcattctgagaatagtgtatgcggcgaccgagttgctcttgcccggcgtca atacgggataataccgcgccacatagcagaactttaaaagtgctcatcattggaaaacgttcttcggggcgaaaactc tcaaggatcttaccgctgttgagatccagttcgatgtaacccactcgtgcacccaactgatcttcagcatcttttactttcac cagcgtttctgggtgagcaaaaacaggaaggcaaaatgccgcaaaaaagggaataagggcgacacggaaatgtt gaatactcatactcttcctttttcaatattattgaagcatttatcagggttattgtctcatgagcggatacatatttgaatgtattt agaaaaataaacaaataggggttccgcgcacatttccccgaaaagtgccacctgcaggaaacagctatgaccatg attacgccaagctctagctagaggtcgacggtatcgagccccagctggttctttccgcctcagaagccatagagccca ccgcatccccagcatgcctgctattgtcttcccaatcctcccccttgctgtcctgccccaccccaccccccagaatagaa tgacacctactcagacaatgcgatgcaatttcctcattttattaggaaaggacagtgggagtggcaccttccagggtca aggaaggcacgggggaggggcaaacaacagatggctggcaactagaaggcacagtcgaggctgatcagcggg tttaaactca-3' (SEQ ID NO. 21);

Gibson assembly technology was used to assemble these four fragments into a pAcC2C9hs-M1-NSP plasmid. The pAcC2C9hs-M1-NSP plasmid was transformed into competent *E*. *coli* DH5α cells, and monoclones were picked for sequencing, and the pAcC2C9hs-M1-NSP plasmid was obtained.

### 1.2 Construction of pAcC2C9hs-M1-G1 plasmid

VEGFA in HEK293T cell genome was selected as the target sequence, and a target sequence DNA of 20 bp in length was selected. The following sequences were synthesized by Sangon Biotech (Shanghai) Co., Ltd.:
Guide1_F: 5'-ATCGAAGTGACAGTATCCTCTGTAT-3' (SEQ ID NO. 22)
Guide1_R: 5'-AAAAATACAGAGGATACTGTCACT-3' (SEQ ID NO. 23)
Phosphorylation and annealing of target sequence DNA: the annealing systems are shown in Table 1 below.

**Table 1**

| Component | Volume |
|---|---|
| 10×T4 DNA ligase Buffer (NEB) | 1 µl |
| Guide-F | 2 µl |
| Guide-R | 2 µl |
| T4 PNK (NEB) | 0.5 µl |
| Sterile enzyme-free water | 4.5 µl |

The phosphorylation procedure involved: reaction at 37°C for 30 minutes, subsequently, NaCl with a final concentration of 50 mM was added to the 10 µl reaction system, followed by slow annealing to obtain an annealed target sequence DNA.

Then, the target sequence DNA was inserted into the pAcC2C9hs-M1-NSP plasmid by Golden Gate assembly to construct a series of pAcC2C9hs-M1-G1 plasmids. The Golden gate assembly system is shown in Table 2 below.

**Table 2**

| Component | Content |
|---|---|
| 10×T4 DNA ligase Buffer (NEB) | 1 µl |
| pAcC2C9hs-M1-NSP | 50 ng |
| Targeted sequence DNA | 0.5 µl |
| T4 DNA ligase (NEB) | 0.5 µl |
| BsaI (NEB) | 0.5 µl |
| Sterile enzyme-free water | To 10 µl |

Ligation procedure: 37°C for 2 min, followed by 16°C for 3 min, repeated for 25 cycles, and finally incubated 80°C for 10 min.

The ligated product was transformed into E. coli DH5α cells, and monoclones were picked for sequencing, the pAcC2C9hs-M1-G1 plasmid (a transient expression plasmid) containing the target sequence was obtained.

### 2. Construction of a series of pAcC2C9hs-M2-M14-G1 plasmids

The plasmid construction was the same as Point 1 (see Construction of pAcC2C9hs-M1-G1 plasmid), except that the expression cassette of the gRNA gRNA_M1 for AcC2C9 in human cells was replaced with the expression cassette of gRNA_M2-gRNA_M14. The 13 optimized versions of the plasmid were named as: pAcC2C9hs-M2-G1, pAcC2C9hs-M3-G1, pAcC2C9hs-M4-G1, pAcC2C9hs-M5-G1, pAcC2C9hs-M6-G1, pAcC2C9hs-M7-G1, pAcC2C9hs-M8-G1, pAcC2C9hs-M9-G1, pAcC2C9hs-M10-G1, pAcC2C9hs-M11-G1, pAcC2C9hs-M12-G1, pAcC2C9hs-M13-G1, and pAcC2C9hs-M14-G1, respectively.

### 3. Gene editing of human cells mediated by pAcC2C9hs-M2-M14-G1 series of plasmids

The activated HEK293T cells were cultured in DMEM medium containing 10% (by volume) FBS. When the cell density reached about 90%, the cells were passaged into a 24-well plate, with approximately 1.0×105 cells per well. After 16-18 hours, 1000 ng of the pAcC2C9hs-M1-M14-G1 plasmids editing different genes was used to transfect the cells using 2 µL of Lipofectamine 3000 (Invitrogen), respectively, in each well. After 24 hours, fresh medium containing puromycin with a final concentration of 2 µg/ml was added for screening. After performing culture for 48 hours, the adherent cells were digested, and genomic DNA was extracted.

The target gene fragment of the target sequence was amplified by PCR, and after gel extraction, the PCR product was annealed using NEBuffer2 (NEB). Subsequently, T7 endonuclease 1 (NEB) was added to the PCR reaction system. followed by enzymatic digestion at 37°C for 15 min, into which 6× Gel Loading Dye (NEB) was then added to terminate the reaction. The reaction products were separated by 6% TBE-PAGE and stained for imaging using 4S Red dye (Sangon Biotech (Shanghai) Co., Ltd.).

4. The gene editing results of the engineered gRNA_M1 to gRNA_M14 for AcC2C9 in mammalian cells are shown in Fig. 2. As shown in the figure, the engineered RNAs for AcC2C9, namely gRNA_M2, gRNA_M3, gRNA_M4, gRNA_M5, gRNA_M6, gRNA_M7, gRNA_M8, gRNA_M9, and gRNA_M10, can improve the gene editing efficiency of AcC2C9 in mammalian cells. gRNA_M9 exhibits the highest efficiency; whereas it can be seen that adding a stabilizing structure at the 3' end cannot improve gene editing efficiency.

### Example 2 Gene editing in mammalian cells using engineered AcC2C9 nuclease gRNA

This example is based on Example 1. In this example, the engineered guide RNA gRNA_M9 (set forth in SEQ ID NO. 12) was selected for AcC2C9. In this example, human embryonic kidney cell HEK293T was used as the experimental cell.

### 1. Construction of a series of pAcC2C9hs-M 1-G plasm ids

In this example, six genes, namely VEGFA, AAVS1, PDCD1, HEXA, EMX1, and TP53 in HEK293T cell genome were selected as the target sequences, and 35 target sequence DNAs of 20 bp in length were selected from these target sequences. The 35 targetsequences comprise G1 described in Example 1. The following sequences were synthesized by Sangon Biotech (Shanghai) Co., Ltd.:
Guide2_F: 5'-ATCGCATGGAAACAGACCTGGCAG-3' (SEQ ID NO. 24)
Guide2_R: 5'-AAAACTGCCAGGTCTGTTTCCATG-3' (SEQ ID NO. 25)
Guide3_F: 5'-ATCGGGATGTGGTGCATTTGGAAT-3' (SEQ ID NO. 26)
Guide3_R: 5'-AAAAATTCCAAATGCACCACATCC-3' (SEQ ID NO. 27)
Guide4_F: 5'-ATCGCGACTCAACCTGGTAAACAT-3' (SEQ ID NO. 28)
Guide4_R: 5'-AAAAATGTTTACCAGGTTGAGTCG-3' (SEQ ID NO. 29)
Guide5_F: 5'-ATCGAATCATTTCCCCAAGAGGAA-3' (SEQ ID NO. 30)
Guide5_R: 5'-AAAATTCCTCTTGGGGAAATGATT-3' (SEQ ID NO. 31)
Guide6_F: 5'-ATCGAGGCAAGCATGGAAACAGAC-3' (SEQ ID NO. 32)
Guide6_R: 5'-AAAAGTCTGTTTCCATGCTTGCCT-3' (SEQ ID NO. 33)
Guide7_F: 5'-ATCGTGTTTCTGCCAGGTCTGTTT-3' (SEQ ID NO. 34)
Guide7_R: 5'-AAAAAAACAGACCTGGCAGAAACA-3' (SEQ ID NO. 35)
Guide8_F: 5'-ATCGAAGGGATGTGGTGCATTTGG-3' (SEQ ID NO. 36)
Guide8_R: 5'-AAAACCAAATGCACCACATCCCTT-3' (SEQ ID NO. 37)
Guide9_F: 5'-ATCGTGTAAGGAAGCTGCAGCACC-3' (SEQ ID NO. 38)
Guide9_R: 5'-AAAAGGTGCTGCAGCTTCCTTACA-3' (SEQ ID NO. 39)
Guide10_F: 5'-ATCGGGAGACATCCGTCGGAGAAG-3' (SEQ ID NO. 40)
Guide10_R: 5'-AAAACTTCTCCGACGGATGTCTCC-3' (SEQ ID NO. 41)
Guide11_F: 5'-ATCGAAGGATGGAGAAAGAGAAAG-3' (SEQ ID NO. 42)
Guide11_R: 5'-AAAACTTTCTCTTTCTCCATCCTT-3' (SEQ ID NO. 43)
Guide12_F: 5'-ATCGAGGAGAAGCAGTTTGGAAAA-3' (SEQ ID NO. 44)
Guide12_R: 5'-AAAATTTTCCAAACTGCTTCTCCT-3' (SEQ ID NO. 45)
Guide13_F: 5'-ATCGCAAACCTTAGAGGTTCTGGC-3' (SEQ ID NO. 46)
Guide13_R: 5'-AAAAGCCAGAACCTCTAAGGTTTG-3' (SEQ ID NO. 47)
Guide14_F: 5'-ATCGGAATCTGCCTAACAGGAGGT-3' (SEQ ID NO. 48)
Guide14_R: 5'-AAAAACCTCCTGTTAGGCAGATTC-3' (SEQ ID NO. 49)
Guide15_F: 5'-ATCGGAGAGAGATGGCTCCAGGAA-3' (SEQ ID NO. 50)
Guide15_R: 5'-AAAATTCCTGGAGCCATCTCTCTC-3' (SEQ ID NO. 51)
Guide16_F: 5'-ATCGAGGATGGAGAGGTGGCTAAA-3' (SEQ ID NO. 52)
Guide16_R: 5'-AAAATTTAGCCACCTCTCCATCCT-3' (SEQ ID NO. 53)
Guide17_F: 5'-ATCGAGCTAGCACAGACTAGAGAG-3' (SEQ ID NO. 54)
Guide17_R: 5'-AAAACTCTCTAGTCTGTGCTAGCT-3' (SEQ ID NO. 55)
Guide18_F: 5'-ATCGGCCATCCTAAGAAACGAGAG-3' (SEQ ID NO. 56)
Guide18_R: 5'-AAAACTCTCGTTTCTTAGGATGGC-3' (SEQ ID NO. 57)
Guide19_F: 5'-ATCGTACCCCGTCTCCCTGGCTTT-3' (SEQ ID NO. 58)
Guide19_R: 5'-AAAAAAAGCCAGGGAGACGGGGTA-3' (SEQ ID NO. 59)
Guide20_F: 5'-ATCGGGGGCAAAGACTGGACCCTG-3' (SEQ ID NO. 60)
Guide20_R: 5'-AAAACAGGGTCCAGTCTTTGCCCC-3' (SEQ ID NO. 61)
Guide21_F: 5'-ATCGCTCGGAGCTGGGACCACGTG-3' (SEQ ID NO. 62)
Guide21_R: 5'-AAAACACGTGGTCCCAGCTCCGAG-3' (SEQ ID NO. 63)
Guide22_F: 5'-ATCGACAGTGGGGACTAGAGCTCA-3' (SEQ ID NO. 64)
Guide22_R: 5'-AAAATGAGCTCTAGTCCCCACTGT-3' (SEQ ID NO. 65)
Guide23_F: 5'-ATCGCATCTTTGCTGTGAGCTCTA-3' (SEQ ID NO. 66)
Guide23_R: 5'-AAAATAGAGCTCACAGCAAAGATG-3' (SEQ ID NO. 67)
Guide24_F: 5'-ATCGATGCTTCAGAGACGAGATGG-3' (SEQ ID NO. 68)
Guide24_R: 5'-AAAACCATCTCGTCTCTGAAGCAT-3' (SEQ ID NO. 69)
Guide25_F: 5'-ATCGGAGCTCTACACCACACCCAA-3' (SEQ ID NO. 70)
Guide25_R: 5'-AAAATTGGGTGTGGTGTAGAGCTC-3' (SEQ ID NO. 71)
Guide26_F: 5'-ATCGCTTAAGCAAATGCCACAGCT-3' (SEQ ID NO. 72)
Guide26_R: 5'-AAAAAGCTGTGGCATTTGCTTAAG-3' (SEQ ID NO. 73)
Guide27_F: 5'-ATCGTTTAACTACTTACTGTTTGT-3' (SEQ ID NO. 74)
Guide27_R: 5'-AAAAACAAACAGTAAGTAGTTAAA-3' (SEQ ID NO. 75)
Guide28_F: 5'-ATCGCTGTGGCATTTGCTTAAGCT-3' (SEQ ID NO. 76)
Guide28_R: 5'-AAAAAGCTTAAGCAAATGCCACAG-3' (SEQ ID NO. 77)
Guide29_F: 5'-ATCGGTTGAACAAACAGTAAGTAG-3' (SEQ ID NO. 78)
Guide29_R: 5'-AAAACTACTTACTGTTTGTTCAAC-3' (SEQ ID NO. 79)
Guide30_F: 5'-ATCGAGAGATGCCTTACTAGGTAC-3' (SEQ ID NO. 80)
Guide30_R: 5'-AAAAGTACCTAGTAAGGCATCTCT-3' (SEQ ID NO. 81)
Guide31_F: 5'-ATCGAGCTTTACAAAAGAAGGGGA-3' (SEQ ID NO. 82)
Guide31_R: 5'-AAAATCCCCTTCTTTTGTAAAGCT-3' (SEQ ID NO. 83)
Guide32_F: 5'-ATCGGACAAGGTTGAACAAACAGT-3' (SEQ ID NO. 84)
Guide32_R: 5'-AAAAACTGTTTGTTCAACCTTGTC-3' (SEQ ID NO. 85)
Guide33_F: 5'-ATCGCAGCTCATTTACAGACGGGA-3' (SEQ ID NO. 86)
Guide33_R: 5'-AAAATCCCGTCTGTAAATGAGCTG-3' (SEQ ID NO. 87)
Guide34_F: 5'-ATCGGTGTCAGTTTAAGAATGGTG-3' (SEQ ID NO. 88)
Guide34_R: 5'-AAAACACCATTCTTAAACTGACAC-3' (SEQ ID NO. 89)
Guide35_F: 5'-ATCGGGTGCAGTTATGCCTCAGAT-3' (SEQ ID NO. 90)
Guide35_R: 5'-AAAAATCTGAGGCATAACTGCACC-3' (SEQ ID NO. 91)

The plasmid construction was the same as Point 1 in Example 1 (see Construction of pAcC2C9hs-M1-G1 plasmid), using the Golden Gate assembly method to insert the annealed double-stranded DNA into the pAcC2C9hs-M1-NSP plasmid to obtain 35 pAcC2C9hs-M1-G plasmids.

### 3. Construction of a series of pAcC2C9hs-M9-G plasmids

The expression cassette of the guide RNA gRNA_M1 for AcC2C9 in human cells was replaced with the expression cassette of the guide gRNA_M9. According to the above method, different target sequences were inserted to obtain 35 pAcC2C9hs-M9-G series of plasmids containing the target sequences.

### 4. Gene editing of human cells mediated by pAcC2C9hs-M9-G series of plasmids

The operation was the same as Point 3 in Example 1 (see Gene editing of human cells mediated by pAcC2C9hs-M2-M14-G1 series of plasmids), except that the pAcC2C9hs-M1-M14-G1 series plasmids in Example 1 were replaced with the pAcC2C9hs-M1-G series plasmids and the pAcC2C9hs-M9-G series plasmids.

### 5. Result

The editing efficiencies of gRNA_M1 and gRNA_M9 in HEK293T cells are shown in Fig. 3. As shown in the figure, using the engineered gRNA_M9 can achieve high-efficiency editing in mammalian cells, wherein an editing efficiency at the same site of gRNA_M9 is approximately 10 times higher than that of gRNA_M1. For example, the editing efficiency at the Guide2 site can be improved from 3% to 30%. The gene editing efficiency of AcC2C9 in mammalian cells has been increased up to 85%, significantly improving the activity and versatility of gene editing of AcC2C9 in mammalian cells. Additionally, no gene editing activity is detected at the Guide6, Guide8, and Guide23 sites when using gRNA_M1, whereas a gene editing efficiency of approximately 10% is detected when using the engineered gRNA_M9. The engineered gRNA_M9 expands the editing scope of the CRISPR/AcC2C9 gene editing system.

The above examples are intended to illustrate the disclosed implementation examples of the present disclosure, and should not be construed as limiting the present disclosure. In addition, various modifications listed herein and changes in methods in the present disclosure are obvious to those skilled in the art without departing from the scope and spirit of the present disclosure. Although the present disclosure has been described in detail with reference to multiple specific preferred examples, it should be understood that the present disclosure should not be limited to these specific examples. In fact, various modifications as mentioned above that are obvious to those skilled in the art to obtain the present disclosure should be included in the scope of the present disclosure.

## Claims

1. A guide RNA, comprising an RNA framework and a gene targeting section,
wherein the RNA framework comprises a tracrRNA and a tracr duplex linked directly or through a linker;
a nucleotide sequence of the tracrRNA is a sequence obtained by addition, deletion, or substitution of one or more nucleotides in a nucleotide sequence set forth in SEQ ID NO. 117, and
a nucleotide sequence of the tracr duplex is a sequence obtained by addition, deletion, or substitution of one or more nucleotides in a nucleotide sequence set forth in SEQ ID NO. 127.

2. The guide RNA according to claim 1, wherein the linker comprises oligonucleotides; preferably, the linker comprises 3-18 nt oligonucleotides; further preferably, a nucleotide sequence of the linker is GAAA.

3. The guide RNA according to claim 1, wherein the gene targeting section further comprises one or more of the following:
1) the gene targeting section is located at a 3' end of the RNA framework;
2) the gene targeting section identifies a PAM sequence on a target gene; preferably, the PAM sequence is 5'-NAAN, wherein N is A, T, C, or G; and
3) the gene targeting section targets a nucleic acid fragment with a length of 12-40 bp downstream of the PAM sequence.

4. The guide RNA according to claim 1, wherein the guide RNA comprises stem-loop structures; preferably, the guide RNA comprises five stem-loop structures, which are stem-loop 2, stem-loop 3, stem-loop 4, stem-loop 5 and stem-loop 6, respectively.

5. The guide RNA according to claim 1, wherein the RNA framework comprises a nucleotide sequence obtained by addition, deletion, or substitution of 15-150 nucleotides at a 5' or 3' end of a nucleotide sequence set forth in SEQ ID NO. 106; or a nucleotide sequence obtained by linking sequences being truncated or substituted 15-150 nucleotides in the middle of the nucleotide sequence set forth in SEQ ID NO. 106 through a linker.

6. The guide RNA according to claim 1, wherein the RNA framework is a gRNA_M2 RNA framework obtained by deletion of 30 bases in stem-loop 1 in the RNA framework with a nucleotide sequence set forth in SEQ ID NO. 106; preferably, a nucleotide sequence of a tracrRNA in the gRNA_M2 RNA framework is set forth in SEQ ID NO. 140, and/or, a nucleotide sequence of a tracr duplex in the gRNA_M2 RNA framework is set forth in SEQ ID NO. 127; more preferably, a nucleotide sequence of the gRNA_M2 RNA framework is set forth in SEQ ID NO. 107.

7. The guide RNA according to claim 1, wherein the RNA framework is a gRNA_M3 RNA framework obtained by deletion of 34 bases in stem-loop 6 in the RNA framework with a nucleotide sequence set forth in SEQ ID NO. 107; preferably, a nucleotide sequence of a tracrRNA in the gRNA_M3 RNA framework is set forth in SEQ ID NO. 118, and/or, a nucleotide sequence of a tracr duplex in the gRNA_M3 RNA framework is set forth in SEQ ID NO. 128; more preferably, a nucleotide sequence of the gRNA_M3 RNA framework is set forth in SEQ ID NO. 108.

8. The guide RNA according to claim 1, wherein the RNA framework is a gRNA_M4 RNA framework obtained by deletion of 43 bases in stem-loop 6 in the RNA framework with a nucleotide sequence set forth in SEQ ID NO. 107; preferably, a nucleotide sequence of a tracrRNA in the gRNA_M4 RNA framework is set forth in SEQ ID NO. 119, and/or, a nucleotide sequence of a tracr duplex in the gRNA_M4 RNA framework is set forth in SEQ ID NO. 129; more preferably, a nucleotide sequence of the gRNA_M4 RNA framework is set forth in SEQ ID NO. 109.

9. The guide RNA according to claim 1, wherein the RNA framework is a gRNA_M5 RNA framework obtained by deletion of 51 bases in stem-loop 6 in the RNA framework with a nucleotide sequence set forth in SEQ ID NO. 107; preferably, a nucleotide sequence of a tracrRNA in the gRNA_M5 RNA framework is set forth in SEQ ID NO. 120, and/or, a nucleotide sequence of a tracr duplex in the gRNA_M5 RNA framework is set forth in SEQ ID NO. 130; more preferably, a nucleotide sequence of the gRNA_M5 RNA framework is set forth in SEQ ID NO. 110.

10. The guide RNA according to claim 1, wherein the RNA framework is a gRNA_M6 RNA framework obtained by deletion of 57 bases in stem-loop 6 in the RNA framework with a nucleotide sequence set forth in SEQ ID NO. 107; preferably, a nucleotide sequence of a tracrRNA in the gRNA_M6 RNA framework is set forth in SEQ ID NO. 121, and/or, a nucleotide sequence of a tracr duplex in the gRNA_M6 RNA framework is set forth in SEQ ID NO. 131; more preferably, a nucleotide sequence of the gRNA_M6 RNA framework is set forth in SEQ ID NO. 111.

11. The guide RNA according to claim 1, wherein the RNA framework is a gRNA_M7 RNA framework obtained by deletion of 64 bases in stem-loop 6 in the RNA framework with a nucleotide sequence set forth in SEQ ID NO. 107; preferably, a nucleotide sequence of a tracrRNA in the gRNA_M7 RNA framework is set forth in SEQ ID NO. 122, and/or, a nucleotide sequence of a tracr duplex in the gRNA_M7 RNA framework is set forth in SEQ ID NO. 132; more preferably, a nucleotide sequence of the gRNA_M7 RNA framework is set forth in SEQ ID NO. 112.

12. The guide RNA according to claim 1, wherein the RNA framework is a gRNA_M8 RNA framework obtained by deletion of 70 bases in stem-loop 6 in the RNA framework with a nucleotide sequence set forth in SEQ ID NO. 107; preferably, a nucleotide sequence of a tracrRNA in the gRNA_M8 RNA framework is set forth in SEQ ID NO. 123, and/or, a nucleotide sequence of a tracr duplex in the gRNA_M8 RNA framework is set forth in SEQ ID NO. 133; more preferably, a nucleotide sequence of the gRNA_M8 RNA framework is set forth in SEQ ID NO. 113.

13. The guide RNA according to claim 1, wherein the RNA framework is a gRNA_M9 RNA framework obtained by deletion of 74 bases in stem-loop 6 in the RNA framework with a nucleotide sequence set forth in SEQ ID NO. 107; preferably, a nucleotide sequence of a tracrRNA in the gRNA_M9 RNA framework is set forth in SEQ ID NO. 124, and/or, a nucleotide sequence of a tracr duplex in the gRNA_M9 RNA framework is set forth in SEQ ID NO. 134; more preferably, a nucleotide sequence of the gRNA_M9 RNA framework is set forth in SEQ ID NO. 114.

14. The guide RNA according to claim 1, wherein the RNA framework is a gRNA_M10 RNA framework obtained by deletion of 78 bases in stem-loop 6 in the RNA framework with a nucleotide sequence set forth in SEQ ID NO. 107; preferably, a nucleotide sequence of a tracrRNA in the gRNA_M10 RNA framework is set forth in SEQ ID NO. 125, and/or, a nucleotide sequence of a tracr duplex in the gRNA_M10 RNA framework is set forth in SEQ ID NO. 135; more preferably, a nucleotide sequence of the gRNA_M10 RNA framework is set forth in SEQ ID NO. 115.

15. The guide RNA according to claim 1, wherein the RNA framework is a gRNA_M11 RNA framework obtained by deletion of 82 bases in stem-loop 6 in the RNA framework with a nucleotide sequence set forth in SEQ ID NO. 107; preferably, a nucleotide sequence of a tracrRNA in the gRNA_M11 RNA framework is set forth in SEQ ID NO. 126, and/or, a nucleotide sequence of a tracr duplex in the gRNA_M11 RNA framework is set forth in SEQ ID NO. 136; more preferably, a nucleotide sequence of the gRNA_M11 RNA framework is set forth in SEQ ID NO. 116.

16. The guide RNA according to claim 1, further comprising an RNA structure stabilizing sequence provided at a 3' end of the guide RNA; preferably, the RNA structure stabilizing sequence is provided at a 3' end of the gene targeting section.

17. The guide RNA according to claim 16, wherein the RNA structure stabilizing sequence is set forth in any one of SEQ ID NOs. 137-139.

18. The guide RNA according to claim 1, wherein a nucleotide sequence of the guide RNA is set forth in any one of SEQ ID NOs. 5-17.

19. An isolated polynucleotide, encoding the guide RNA according to any one of claims 1 to 18.

20. A construct, comprising the isolated polynucleotide according to claim 19.

21. An expression system, comprising the construct according to claim 20 or a genome exogenously being introduced the polynucleotide according to claim 19.

22. A gene editing system, comprising the guide RNA according to any one of claims 1 to 18 or the polynucleotide according to claim 19.

23. The gene editing system according to claim 22, further comprising a nuclease or a polynucleotide encoding the same.

24. The gene editing system according to claim 23, wherein the nuclease is a CRISPR nuclease; preferably, the nuclease is selected from Cas9, Cas12 and Cas13 protein families and variants thereof; further preferably, the Cas nuclease is selected from nSpCas9 and a variant thereof, SaCas9 and a variant thereof, Cas12a and a variant thereof, and C2C9 and a variant thereof; more preferably, the Cas nuclease is an AcC2C9 nuclease or a variant thereof.

25. The gene editing system according to claim 24, wherein the AcC2C9 nuclease comprises at least one of the following:
(I) a wild-type AcC2C9 nuclease or a fragment thereof; wherein the AcC2C9 nuclease is derived from *Actinomadura craniellae* C2C9, with an amino acid sequence set forth in SEQ ID NO. 1; a human codon-optimized nucleic acid sequence of the AcC2C9 nuclease is set forth in SEQ ID NO. 3;
or (II) except for (I), the AcC2C9 nuclease further comprises a nuclear localization signal fragment.

26. A pharmaceutical composition, comprising the gene editing system according to any one of claims 22 to 24, and a pharmaceutically acceptable carrier.

27. A gene editing method, comprising contacting a target gene with the gene editing system according to any one of claims 22 to 24 to realize editing of the target gene.

28. The gene editing method according to claim 27, comprising the following:
i) introducing the AcC2C9 nuclease or the polynucleotide encoding the same and the guide RNA or the polynucleotide encoding the same into a cell;
ii) under the mediation of the AcC2C9 nuclease, producing one or more nicks in the target gene, or targeting, editing, modifying or manipulating the target gene.

29. The gene editing method according to claim 27 or 28, comprising the following:
1) guiding the AcC2C9 nuclease to the target gene by the guide RNA according to any one of claims 1 to 18;
2) forming a complex comprising the AcC2C9 nuclease and the guide RNA according to any one of claims 1 to 18, and recognizing a PAM sequence on the target gene; and
3) introducing a donor template comprising a heterologous polynucleotide sequence into the cell.

30. Use of the guide RNA according to any one of claims 1 to 18, the isolated polynucleotide according to claim 19, the construct according to claim 20, the expression system according to claim 21, the gene editing system according to any one of claims 22 to 25, the pharmaceutical composition according to claim 26 or the method according to any one of claims 27 to 29 in gene editing of a target gene and/or a related polypeptide in an *in vivo, ex vivo* cell or cell-free environment.

31. The use according to claim 30, wherein the gene editing is selected from gene cleavage, gene deletion, gene insertion, point mutation, transcription repression, transcription activation and/or base editing.

32. A cell, obtained by gene editing with the gene editing system according to any one of claims 21 to 24 or the method according to any one of claims 26 to 28.

33. A method for preparing a guide RNA, comprising modification of an RNA framework of an original guide RNA, and the modification is selected from modification of tracrRNA or crRNA in the RNA framework alone or modification of tracrRNA and crRNA in the RNA framework simultaneously, and the modification is addition, deletion, or substitution of one or more nucleotides in a nucleotide of a tracrRNA and/or a crRNA, or linking the tracrRNA and the crRNA through a linker.
